# EUROPEAN PATENT APPLICATION

(11) **EP 2 386 634 A1**
(43) Date of publication of application: **16.11.2011**
(21) Application number: 09837482.0
(22) Date of filing: 07.01.2009
(51) Int. Cl.: C12N 15/09, C07K 19/00, C12N 1/21, C12N 9/06, C12P 21/02, C12P 33/16

(54) **STEROL SIDE CHAIN-CLEAVING ENZYME PROTEIN AND USE THEREOF**

(71) Applicant: Mitsubishi Chemical Corporation, Tokyo 108-0014 (JP)
(72) Inventor: YAMAGISHI, Masahiro, Yokohama-shi Kanagawa 227-8502 (JP); SAKAMOTO, Takeshi, Yokohama-shi Kanagawa 227-8502 (JP); UEDA, Makoto, Yokohama-shi Kanagawa 227-8502 (JP); ITOH, Masashi, Fukuroi-shi Shizuoka 437-0023 (JP); FUJII, Yoshikazu, Iwata-shi Shizuoka 438-0812 (JP); KABUMOTO, Hiroki, Kawasaki-shi Kanagawa 210-0802 (JP); ARISAWA, Akira, Iwata-shi Shizuoka 438-0078 (JP); FUJII, Tadashi, Iwata-shi Shizuoka 438-0078 (JP)
(74) Representative: Polypatent
(86) International application number: PCT/JP2009/050095
(87) International publication number: WO 2010/079594

(57) **Abstract**

It is an object of the present invention to obtain highly active P450scc enzyme protein which is is an important enzyme protein that catalyzes the first step of the biosynthesis of industrially useful steroid hormone. The present invention provides a sterol side chain cleavage enzyme protein having the following physicochemical properties:
(1) action: the enzyme acts on sterol represented by formula (I) as defeined in the specifciatin and cleaves the carbon-carbon bond between positions 20 and 22 of a sterol side chain portion by its activity of cleaving the bonds, so as to generate a compound represented by formula (II) as defeined in the specifciatin;
(2) substrate specificity: when microorganisms that produce the enzyme protein are allowed to react with an aqueous solution containing 100 µg/ml 4-cholesten-3-onc or cholesterol at 28°C for 5 hours, the conversion reaction rate from 4-cholesten-3-one to progesterone is 10% or more, and the conversion rate from cholesterol to pregnenolone is 10% or more;

SEQUENCE LISTING
<110> Mitsubishi Chemical Corporation
<120> An enzyme protein of cleaving sterol side chain and use thereof
<130> A8084
<160> 23
<210> 1
<211> 1419
<212> DNA
<213> Sphingomonas subterranea
<220>
<221> CDS
<222> (1)..(1416)
<400> 1
atg gca agt gca gca gcg ggt gca gac ggc ctt ccc ttg ctc gat ggc 48
Met Ala Ser Ala Ala Ala Gly Ala Asp Gly Leu Pro Leu Leu Asp Gly
1               5                   10                  15
ggc gtg ccg ctg ctc ggg cac ctc gcc cag ttc ttt cgc gat ccg gtt 96
Gly Val Pro Leu Leu Gly His Leu Ala Gln Phe Phe Arg Asp Pro Val
            20                  25                  30
tcc gtc ctg aag cgc ggc tat cgc tcg aag ggc cgg ctc ttc gcg atg 144
Ser Val Leu Lys Arg Gly Tyr Arg Ser Lys Gly Arg Leu Phe Ala Met
        35                  40                  45
aac ttc atg ggc cag cgc atg aac gtg atg ctg ggg ccg gaa cac aac 192
Asn Phe Met Gly Gln Arg Met Asn Val Met Leu Gly Pro Glu His Asn
    50                  55                  60
cgc ttc ttc ttc gag gaa acc gac aag ctg ctc tcg atc cga gag tcg 240
Arg Phe Phe Phe Glu Glu Thr Asp Lys Leu Leu Ser Ile Arg Glu Ser
65                  70                  75                  80
atg ccg ttc ttc ctc aag atg ttc tcg ccc gag ttc tac tcg ttt gcc 288
Met Pro Phe Phe Leu Lys Met Phe Ser Pro Glu Phe Tyr Ser Phe Ala
                85                  90                  95
gag atg gac gaa tac ctg cgc cag cgc gcg atc atc atg ccc cgg ttc 336
Glu Met Asp Glu Tyr Leu Arg Gln Arg Ala Ile Ile Met Pro Arg Phe
            100                 105                 110
aag gcg gca tcg atg aag cag tac gtg ccg gtg atg gtc gag gaa tcg 384
Lys Ala Ala Ser Met Lys Gln Tyr Val Pro Val Met Val Glu Glu Ser
        115                 120                 125
ctg aac ctc gtc gaa cgg ctg ggt gag gaa ggc gag ttc gac ctg atc 432
Leu Asn Leu Val Glu Arg Leu Gly Glu Glu Gly Glu Phe Asp Leu Ile
    130                 135                 140
ccg acg ctg ggc ccc gtg gta atg gac atc gcc gcg cac agc ttc atg 480
Pro Thr Leu Gly Pro Val Val Met Asp Ile Ala Ala His Ser Phe Met
145                 150                 155                 160
ggg cgc gaa ttc cac gag aag ctt ggc cac gaa ttc ttc gag ctg ttc 528
Gly Arg Glu Phe His Glu Lys Leu Gly His Glu Phe Phe Glu Leu Phe
                165                 170                 175
cgc gac ttc tcg ggc ggc atg gag ttc gtg ctg ccg ctg tgg ctg ccg 576
Arg Asp Phe Ser Gly Gly Met Glu Phe Val Leu Pro Leu Trp Leu Pro
            180                 185                 190
aca cca aag atg gtg aaa agc cag cgc gcc aag aag aag ctc cac gcg 624
Thr Pro Lys Met Val Lys Ser Gln Arg Ala Lys Lys Lys Leu His Ala
        195                 200                 205
atc ctg caa tcg tgg atc gac aag cgc cgc gcc agt ccg ctc gat ccg 672
Ile Leu Gln Ser Trp Ile Asp Lys Arg Arg Ala Ser Pro Leu Asp Pro
    210                 215                 220
ccc gac ttc ttc cag acg atg atc gag acg aag tat ccc gat ggc cgc 720
Pro Asp Phe Phe Gln Thr Met Ile Glu Thr Lys Tyr Pro Asp Gly Arg
225                 230                 235                 240
gcc gtg ccc gac gag atc atc cgg cac ctg atc ctg ctg ctg gtc tgg 768
Ala Val Pro Asp Glu Ile Ile Arg His Leu Ile Leu Leu Leu Val Trp

                  245                 250                 255
gcg ggc cac gaa acc acc gcc ggg caa gtc agc tgg gcg ctg gcc gac       816
Ala Gly His Glu Thr Thr Ala Gly Gln Val Ser Trp Ala Leu Ala Asp
            260                 265                 270
ctc ctc cag aac ccc ggc tac cag cag gtg ctg cgc gaa gag gtc gcc       864
Leu Leu Gln Asn Pro Gly Tyr Gln Gln Val Leu Arg Glu Glu Val Ala
        275                 280                 285
tcg ctg ctc ggc ggc agc gac ggg cgc gca ctg ggc tgg gag cag gcc       912
Ser Leu Leu Gly Gly Ser Asp Gly Arg Ala Leu Gly Trp Glu Gln Ala
    290                 295                 300
gtg gcg atg gag aag atg gac ctc gcc ctg cgc gag aca gag cgg ctc       960
Val Ala Met Glu Lys Met Asp Leu Ala Leu Arg Glu Thr Glu Arg Leu
305                 310                 315                 320
cac ccc gtc gcc tac atg ctc agc cgc aag gcc agt gcc gat atc gag      1008
His Pro Val Ala Tyr Met Leu Ser Arg Lys Ala Ser Ala Asp Ile Glu
                325                 330                 335
cgc gac ggc tat cgc atc cgc aag ggc gaa ttc gtg ctg ctc gcg ccc      1056
Arg Asp Gly Tyr Arg Ile Arg Lys Gly Glu Phe Val Leu Leu Ala Pro
            340                 345                 350
tcg gtc agc cat cgc atg gaa gaa acc ttc cgc aat gcc gat gcc tat      1104
Ser Val Ser His Arg Met Glu Glu Thr Phe Arg Asn Ala Asp Ala Tyr
        355                 360                 365
gac ccc gag cgc ttc aac ccg cag aac ccg gac gcg cag atc gag agc      1152
Asp Pro Glu Arg Phe Asn Pro Gln Asn Pro Asp Ala Gln Ile Glu Ser
    370                 375                 380
aac tcg ctg atc ggc ttc ggc ggc ggg gtg cat cgc tgt gcg ggg gta      1200
Asn Ser Leu Ile Gly Phe Gly Gly Gly Val His Arg Cys Ala Gly Val
385                 390                 395                 400
aac ttc gcg cgc atg gaa atg aag gtg ctg gtg gcg atc ctg ctg cag      1248
Asn Phe Ala Arg Met Glu Met Lys Val Leu Val Ala Ile Leu Leu Gln
                405                 410                 415
aac ttc gac atg gag ctg atc gac gaa gtg cgc ccc atc gcc ggc gca      1296
Asn Phe Asp Met Glu Leu Ile Asp Glu Val Arg Pro Ile Ala Gly Ala
            420                 425                 430
tcg acc tac tgg ccc gcc cag ccc tgc cgc gtg cgc tac aag cgg cgc      1344
Ser Thr Tyr Trp Pro Ala Gln Pro Cys Arg Val Arg Tyr Lys Arg Arg
        435                 440                 445
aag ctg gat ggc gcg ggt gga agc gcg gac gtg gcg gcg ctc gcc aag      1392
Lys Leu Asp Gly Ala Gly Gly Ser Ala Asp Val Ala Ala Leu Ala Lys
    450                 455                 460
gcg gcc ggt tgc ccg gcc cat gca tga                                  1419
Ala Ala Gly Cys Pro Ala His Ala
465                 470
<210>  2
<211>  472
<212>  PRT
<213>  Sphingomonas subterranea
<400>  2
Met Ala Ser Ala Ala Ala Gly Ala Asp Gly Leu Pro Leu Leu Asp Gly
1               5                   10                  15
Gly Val Pro Leu Leu Gly His Leu Ala Gln Phe Phe Arg Asp Pro Val
            20                  25                  30
Ser Val Leu Lys Arg Gly Tyr Arg Ser Lys Gly Arg Leu Phe Ala Met
        35                  40                  45
Asn Phe Met Gly Gln Arg Met Asn Val Met Leu Gly Pro Glu His Asn
    50                  55                  60
Arg Phe Phe Phe Glu Glu Thr Asp Lys Leu Leu Ser Ile Arg Glu Ser
65                  70                  75                  80
Met Pro Phe Phe Leu Lys Met Phe Ser Pro Glu Phe Tyr Ser Phe Ala
                85                  90                  95

Glu Met Asp Glu Tyr Leu Arg Gln Arg Ala Ile Ile Met Pro Arg Phe
            100                 105                 110
Lys Ala Ala Ser Met Lys Gln Tyr Val Pro Val Met Val Glu Glu Ser
        115                 120                 125
Leu Asn Leu Val Glu Arg Leu Gly Glu Glu Gly Glu Phe Asp Leu Ile
    130                 135                 140
Pro Thr Leu Gly Pro Val Val Met Asp Ile Ala Ala His Ser Phe Met
145                 150                 155                 160
Gly Arg Glu Phe His Glu Lys Leu Gly His Glu Phe Phe Glu Leu Phe
                165                 170                 175
Arg Asp Phe Ser Gly Gly Met Glu Phe Val Leu Pro Leu Trp Leu Pro
            180                 185                 190
Thr Pro Lys Met Val Lys Ser Gln Arg Ala Lys Lys Lys Leu His Ala
        195                 200                 205
Ile Leu Gln Ser Trp Ile Asp Lys Arg Arg Ala Ser Pro Leu Asp Pro
    210                 215                 220
Pro Asp Phe Phe Gln Thr Met Ile Glu Thr Lys Tyr Pro Asp Gly Arg
225                 230                 235                 240
Ala Val Pro Asp Glu Ile Ile Arg His Leu Ile Leu Leu Leu Val Trp
                245                 250                 255
Ala Gly His Glu Thr Thr Ala Gly Gln Val Ser Trp Ala Leu Ala Asp
            260                 265                 270
Leu Leu Gln Asn Pro Gly Tyr Gln Gln Val Leu Arg Glu Glu Val Ala
        275                 280                 285
Ser Leu Leu Gly Gly Ser Asp Gly Arg Ala Leu Gly Trp Glu Gln Ala
    290                 295                 300
Val Ala Met Glu Lys Met Asp Leu Ala Leu Arg Glu Thr Glu Arg Leu
305                 310                 315                 320
His Pro Val Ala Tyr Met Leu Ser Arg Lys Ala Ser Ala Asp Ile Glu
                325                 330                 335
Arg Asp Gly Tyr Arg Ile Arg Lys Gly Glu Phe Val Leu Leu Ala Pro
            340                 345                 350
Ser Val Ser His Arg Met Glu Glu Thr Phe Arg Asn Ala Asp Ala Tyr
        355                 360                 365
Asp Pro Glu Arg Phe Asn Pro Gln Asn Pro Asp Ala Gln Ile Glu Ser
    370                 375                 380
Asn Ser Leu Ile Gly Phe Gly Gly Gly Val His Arg Cys Ala Gly Val
385                 390                 395                 400
Asn Phe Ala Arg Met Glu Met Lys Val Leu Val Ala Ile Leu Leu Gln
                405                 410                 415
Asn Phe Asp Met Glu Leu Ile Asp Glu Val Arg Pro Ile Ala Gly Ala
            420                 425                 430
Ser Thr Tyr Trp Pro Ala Gln Pro Cys Arg Val Arg Tyr Lys Arg Arg
        435                 440                 445
Lys Leu Asp Gly Ala Gly Gly Ser Ala Asp Val Ala Ala Leu Ala Lys
    450                 455                 460
Ala Ala Gly Cys Pro Ala His Ala
465                 470
<210>  3
<211>  1762
<212>  DNA
<213>  Sphingomonas subterranea
<400>  3
atggcaagtg cagcagcggg tgcagacggc cttcccttgc tcgatggcgg cgtgccgctg      60
ctcgggcacc tcgcccagtt ctttcgcgat ccggtttccg tcctgaagcg cggctatcgc     120
tcgaagggcc ggctcttcgc gatgaacttc atgggccagc gcatgaacgt gatgctgggg     180
ccggaacaca accgcttctt cttcgaggaa accgacaagc tgctctcgat ccgagagtcg     240
atgccgttct tcctcaagat gttctcgccc gagttctact cgtttgccga gatggacgaa     300
tacctgcgcc agcgcgcgat catcatgccc cggttcaagg cggcatcgat gaagcagtac     360
gtgccggtga tggtcgagga atcgctgaac ctcgtcgaac ggctgggtga ggaaggcgag     420

ttcgacctga tcccgacgct gggccccgtg gtaatggaca tcgccgcgca cagcttcatg 480
gggcgcgaat tccacgagaa gcttggccac gaattcttcg agctgttccg cgacttctcg 540
ggcggcatgg agttcgtgct gccgctgtgg ctgccgacac caaagatggt gaaaagccag 600
cgcgccaaga agaagctcca cgcgatcctg caatcgtgga tcgacaagcg ccgcgccagt 660
ccgctcgatc cgcccgactt cttccagacg atgatcgaga cgaagtatcc cgatggccgc 720
gccgtgcccg acgagatcat ccggcacctg atcctgctgc tggtctgggc gggccacgaa 780
accaccgccg ggcaagtcag ctgggcgctg gccgacctcc tccagaaccc cggctaccag 840
caggtgctgc gcgaagaggt cgcctcgctg ctcggcggca gcgacgggcg cgcactgggc 900
tgggagcagg ccgtggcgat ggagaagatg gacctcgccc tgcgcgagac agagcggctc 960
caccccgtcg cctacatgct cagccgcaag gccagtgccg atatcgagcg cgacggctat 1020
cgcatccgca agggcgaatt cgtgctgctc gcgccctcgg tcagccatcg catggaagaa 1080
accttccgca atgccgatgc ctatgacccc gagcgcttca acccgcagaa cccggacgcg 1140
cagatcgaga gcaactcgct gatcggcttc ggcggcgggg tgcatcgctg tgcgggggta 1200
aacttcgcgc gcatggaaat gaaggtgctg gtggcgatcc tgctgcagaa cttcgacatg 1260
gagctgatcg acgaagtgcg ccccatcgcc ggcgcatcga cctactggcc cgcccagccc 1320
tgccgcgtgc gctacaagcg gcgcaagctg gatggcgcgg gtggaagcgc ggacgtggcg 1380
gcgctcgcca aggcggccgg ttgcccggcc catgcatgag gcgcactgaa tggccaaggt 1440
gactttcgtc cagccggacg gctcggcgcg gacttgcgtg aacttcgagg gcatgaccct 1500
gatgcagctc gcggtcggca accttgtcga cgggatcgac gcgctgtgcg gcggcatgat 1560
gcagtgcgcc acctgccatt gctacatcga cccggactgg cttgaccgca ccggccccgc 1620
ccggccggaa gaacgcgaga tgctcgaggc catcgatggc gtcgagatcc gcccgaacag 1680
ccgcctgtcc tgccaggtcc agcttggcga ggagttggac gggctggtgg tgcacattcc 1740
ggcggagcaa ccgggagttt ag 1762
<210> 4
<211> 1765
<212> DNA
<213> Novosphingobium aromaticivorans
<400> 4
atggcaagag ctgcgactgc ggccggtaat ggccttccct tgctcgatgg aggcgtgccg 60
ctcctcgggc atctcgcaca gttcttccgc gatccggttt cggtactcaa gcgcggatac 120
cgctcgaagg ggcggctctt cgcgatgaac ttcatgggcc agcgcatgaa cgtgatgctg 180
ggtccggaac acaaccgctt cttcttcgag gagacggaca agctgctctc gatccgggag 240
tcgatgccgt tcttcctcaa gatgttctcg cccgagttct attcgttcgc ggaaatggac 300
gagtacctgc gccagcgctc gatcatcatg ccccgcttca aggcggcatc gatgaagcag 360
tacgtgccgg tcatggtcga ggaatcgctt aacctggtcg agcggctggg cgaggaaggc 420
gagttcgacc tgatcccgac gctgggcccg gtggtaatgg acatcgccgc gcacagcttc 480
atgggacgcg agttccacga gaagctgggg catgagttct tcgaactctt ccgcgatttt 540
tcgggaggca tggaattcgt cctgccgctg tggctgccga cacccaagat ggtcaagtca 600
cagcgcgcga agaggaagct ccacgccatc ctgcaatcgt ggatcgacaa gcgccgcgcc 660
gccccgctcg atccgcccga tttcttccag acgatgatcg agacgaagta tcccgatggc 720
cgcccggtgc ccgacgagat catccgccac ctgatcctcc ttctcgtctg ggcagggcac 780
gagacgaccg ccgggcaggt gagctgggcg ctggcggacc tccttcagaa cccggactac 840
cagaaggtgc tgcgcggcga gatatcgtcg ctgctgggcg gcagcgacgg gcgcgacctt 900
ggctgggaac aggccgtggc gatggagaag atggaccttg ccctgcgcga gaccgagcgg 960
ctccatccgg tcgcctacat gctcagccgc aaggcgcggg ccgatatcga gcgcgacggc 1020
tatgtcatcc gcaagggcga gttcgtgctg cttgcgcctt cggtcagcca ccgcatggaa 1080
gagacgttcc gcaatcccga tgcctatgac ccggaacgct tcaacccggc caaccccgat 1140
gcgcagatcg aaagcaattc gttgatcggc tttggcgggg gtgtccaccg ctgcgcgggc 1200
gtgaacttcg cgcggatgga gatgaaggtg ctggtggcga tcctgctcca gaacttcgac 1260
atggagctga tggacgaagt gcggcccatc gcgggcgcat cgacctactg gcccgcccag 1320
ccctgccggg tgcgctatcg gcggcgcaag ctcgacgggt cggaggcagg tgcggacatg 1380
gcggcgctgg cccgagccgc cggctgcccg gcgcatacgt gagggaggcc tgatggccaa 1440
ggtgactttc gtccagccgg acggatcgca gcgaacctgc gtgaacttcg aaggcatgac 1500
gttgatgcag ctcgcagtgg gcaatctcgt cgacgggatc gacgcgctgt gcggcggcat 1560
gatgcagtgc gcgacctgcc attgctggat cgaccccgaa tggatcggcc gcaccggcat 1620
ggccggaccc gatgagcggg caatgctgga agcgatcgag ggcgtcgaga tccgtcccga 1680
aagccgcctg tcctgccagg tacagcttgg cgaagaactt gacggctggg tcgtgcgcat 1740
tccaccggag caaccgggag tttag 1765
<210> 5
<211> 32

<212> DNA
<213> Artificial Sequence
<220>
<223> Description of Artificial Sequence: Synthetic DNA
<400> 5
cgagatctga tgaacttcat gggccagcgc at       32
<210> 6
<211> 32
<212> DNA
<213> Artificial Sequence
<220>
<223> Description of Artificial Sequence: Synthetic DNA
<400> 6
cgagatctgc agcggtggac acccccgcca aa       32
<210> 7
<211> 35
<212> DNA
<213> Artificial Sequence
<220>
<223> Description of Artificial Sequence: Synthetic DNA
<400> 7
ccccatatgg caagtgcagc agcgggtgca gacgg    35
<210> 8
<211> 29
<212> DNA
<213> Artificial Sequence
<220>
<223> Description of Artificial Sequence: Synthetic DNA
<400> 8
gcactagtct aaactcccgg ttgctccgg           29
<210> 9
<211> 30
<212> DNA
<213> Artificial Sequence
<220>
<223> Description of Artificial Sequence: Synthetic DNA
<400> 9
gcccatatgg caagagctgc gactgcggcc          30
<210> 10
<211> 29
<212> DNA
<213> Artificial Sequence
<220>
<223> Description of Artificial Sequence: Synthetic DNA
<400> 10
gcactagtct aaactcccgg ttgctccgg           29
<210> 11
<211> 29
<212> DNA
<213> Artificial Sequence
<220>
<223> Description of Artificial Sequence: Synthetic DNA
<400> 11
ccgctagcgc aagagctgcg actgcggcc           29
<210> 12
<211> 29
<212> DNA
<213> Artificial Sequence
<220>
<223> Description of Artificial Sequence: Synthetic DNA

<400> 12
gcggatccct aaactcccgg ttgctccgg          29
<210> 13
<211> 30
<212> DNA
<213> Artificial Sequence
<220>
<223> Description of Artificial Sequence: Synthetic DNA
<400> 13
gcccatatgg caagagctgc gactgcggcc          30
<210> 14
<211> 30
<212> DNA
<213> Artificial Sequence
<220>
<223> Description of Artificial Sequence: Synthetic DNA
<400> 14
gccctcgagt cacgtatgcg ccgggcagcc          30
<210> 15
<211> 30
<212> DNA
<213> Artificial Sequence
<220>
<223> Description of Artificial Sequence: Synthetic DNA
<400> 15
gcccatatgg caagtgcagc agcgggtgca          30
<210> 16
<211> 30
<212> DNA
<213> Artificial Sequence
<220>
<223> Description of Artificial Sequence: Synthetic DNA
<400> 16
gccctcgagt catgcatggg ccgggcaacc          30
<210> 17
<211> 30
<212> DNA
<213> Artificial Sequence
<220>
<223> Description of Artificial Sequence: Synthetic DNA
<400> 17
gcccatatgg cttcaacacg tagtcctcgc          30
<210> 18
<211> 30
<212> DNA
<213> Artificial Sequence
<220>
<223> Description of Artificial Sequence: Synthetic DNA
<400> 18
gccctcgagt cactgctggg ttgcttcctg          30
<210> 19
<211> 45
<212> DNA
<213> Artificial Sequence
<220>
<223> Description of Artificial Sequence: Synthetic DNA
<400> 19
ccgcatatgg cttcaacacg tagtcctcgc cccttcaatg agatc     45
<210> 20
<211> 35

<212> DNA
<213> Artificial Sequence
<220>
<223> Description of Artificial Sequence: Synthetic DNA
<400> 20
gcactagttc actgctgggt tgcttcctgg ttaaa    35
<210> 21
<211> 45
<212> DNA
<213> Artificial Sequence
<220>
<223> Description of Artificial Sequence: Synthetic DNA
<400> 21
gcactagtaa ggaaacagac catgtccaca caggagcaga ccccc     45
<210> 22
<211> 29
<212> DNA
<213> Artificial Sequence
<220>
<223> Description of Artificial Sequence: Synthetic DNA
<400> 22
cgtgatcagg catcagacac ggcatcagg          29
<210>  23
<211>  473
<212>  PRT
<213>  Novosphingobium aromaticivorans
<400>  23
Met Ala Arg Ala Ala Thr Ala Ala Gly Asn Gly Leu Pro Leu Leu Asp
1               5                   10                  15
Gly Gly Val Pro Leu Leu Gly His Leu Ala Gln Phe Phe Arg Asp Pro
            20                  25                  30
Val Ser Val Leu Lys Arg Gly Tyr Arg Ser Lys Gly Arg Leu Phe Ala
        35                  40                  45
Met Asn Phe Met Gly Gln Arg Met Asn Val Met Leu Gly Pro Glu His
    50                  55                  60
Asn Arg Phe Phe Phe Glu Glu Thr Asp Lys Leu Leu Ser Ile Arg Glu
65                  70                  75                  80
Ser Met Pro Phe Phe Leu Lys Met Phe Ser Pro Glu Phe Tyr Ser Phe
                85                  90                  95
Ala Glu Met Asp Glu Tyr Leu Arg Gln Arg Ser Ile Ile Met Pro Arg
            100                 105                 110
Phe Lys Ala Ala Ser Met Lys Gln Tyr Val Pro Val Met Val Glu Glu
        115                 120                 125
Ser Leu Asn Leu Val Glu Arg Leu Gly Glu Glu Gly Glu Phe Asp Leu
    130                 135                 140
Ile Pro Thr Leu Gly Pro Val Val Met Asp Ile Ala Ala His Ser Phe
145                 150                 155                 160
Met Gly Arg Glu Phe His Glu Lys Leu Gly His Glu Phe Phe Glu Leu
                165                 170                 175
Phe Arg Asp Phe Ser Gly Gly Met Glu Phe Val Leu Pro Leu Trp Leu
            180                 185                 190
Pro Thr Pro Lys Met Val Lys Ser Gln Arg Ala Lys Arg Lys Leu His
        195                 200                 205
Ala Ile Leu Gln Ser Trp Ile Asp Lys Arg Arg Ala Ala Pro Leu Asp
    210                 215                 220
Pro Pro Asp Phe Phe Gln Thr Met Ile Glu Thr Lys Tyr Pro Asp Gly
225                 230                 235                 240
Arg Pro Val Pro Asp Glu Ile Ile Arg His Leu Ile Leu Leu Leu Val
                245                 250                 255
Trp Ala Gly His Glu Thr Thr Ala Gly Gln Val Ser Trp Ala Leu Ala

            260                 265                 270
Asp Leu Leu Gln Asn Pro Asp Tyr Gln Lys Val Leu Arg Gly Glu Ile
        275                 280                 285
Ser Ser Leu Leu Gly Gly Ser Asp Gly Arg Asp Leu Gly Trp Glu Gln
    290                 295                 300
Ala Val Ala Met Glu Lys Met Asp Leu Ala Leu Arg Glu Thr Glu Arg
305                 310                 315                 320
Leu His Pro Val Ala Tyr Met Leu Ser Arg Lys Ala Arg Ala Asp Ile
                325                 330                 335
Glu Arg Asp Gly Tyr Val Ile Arg Lys Gly Glu Phe Val Leu Leu Ala
            340                 345                 350
Pro Ser Val Ser His Arg Met Glu Glu Thr Phe Arg Asn Pro Asp Ala
        355                 360                 365
Tyr Asp Pro Glu Arg Phe Asn Pro Ala Asn Pro Asp Ala Gln Ile Glu
    370                 375                 380
Ser Asn Ser Leu Ile Gly Phe Gly Gly Gly Val His Arg Cys Ala Gly
385                 390                 395                 400
Val Asn Phe Ala Arg Met Glu Met Lys Val Leu Val Ala Ile Leu Leu
                405                 410                 415
Gln Asn Phe Asp Met Glu Leu Met Asp Glu Val Arg Pro Ile Ala Gly
            420                 425                 430
Ala Ser Thr Tyr Trp Pro Ala Gln Pro Cys Arg Val Arg Tyr Arg Arg
        435                 440                 445
Arg Lys Leu Asp Gly Ser Glu Ala Gly Ala Asp Met Ala Ala Leu Ala
    450                 455                 460
Arg Ala Ala Gly Cys Pro Ala His Thr
465                 470

## Description

### Technical Field

The present invention relates to: an enzyme protein that cleaves the bond between positions 20 and 22 of a sterol side chain to produce pregnenolone and the like that are industrially useful as medicaments or pharmaceutical intermediates; DNA encoding the enzyme protein; a transformant obtained by introducing the DNA into a vector; a method for producing pregnenolone and the like, and hydrocortisone and a derivative thereof, using the enzyme protein; etc.

### Background Art

11β,17α,21-trihydroxy-4-pregnen-3,20-dione (hydrocortisone), and its precursor substances, pregnenolone, progesterone, and 7-dehydropregnenolone, are compounds that are industrially useful as medicaments and pharmaceutical intermediates. As a conventional method for producing pregnenolone, naturally-occurring sterol compounds such as cholesterol, diosgenin and stigmasterol are used as raw materials, and such pregnenolone is produced by a plurality of organic synthetic reactions (Non-Patent Document 1). However, these methods are problematic as industrial production methods, in that they include many steps and the yield of the product of interest is low. On the other hand, as a method for biochemically producing hydrocortisone, the sterol conversion method, which uses the enzyme (side chain cleavage cytochrome P450; hereinafter referred to as "P450scc" at times) having activity of cleaving the bond between positions 20 and 22 of the sterol side chain derived from an animal, has been disclosed (Non-Patent Document 2). However, such an animal-derived enzyme protein has low activity. In addition, the culture of cells to be used as host cells requires an expensive medium, and a proliferation rate is low. Thus, this production method has not yet been sufficiently established. Moreover, there has also been disclosed a method comprising introducing bovine-derived P450scc into the yeast and then converting cholesterol to pregnenolone in the presence of adrenodoxin and adrenodoxin reductase that constitute the electron transfer system. However, productivity is extremely low, and thus, productivity at an industrial level has not yet been obtained (Patent Document 1).
[Non-Patent Document 1] J. Org. Chem., 1979, 44, 1583
[Non-Patent Document 2] Proc. Natl. Acad. Sci. USA, 1988, 85,1988
[Patent Document 1] Japanese Patent No. 2963711

### Disclosure of the Invention

### Problems to be Solved by the Invention

It is an object to be achieved by the present invention to provide highly active P450scc that catalyzes the first step of the biosynthesis of industrially useful steroid hormone, and a method for simply producing pregnenolone and the like, and further, hydrocortisone, at low costs, using the aforementioned P450scc.

### Means for Solving the Problems

As a result of intensive studies directed towards achieving the aforementioned object, the present inventors have measured several hundreds of P450scc from microorganisms, in terms of the activity of cleaving the bond between positions 20 and 22 of a sterol side chain, and have discovered an enzyme protein having extremely high activity of cleaving the bond between positions 20 and 22 of a sterol compound side chain. The inventors have discovered for the first time that this protein has the aforementioned activity. Moreover, the inventors have screened for DNAs encoding the P450 proteins of the closely-related microorganisms, using, as an indicator, high homology at the nucleotide sequence level to the aforementioned microorganism-derived enzyme protein. As a result, they have discovered DNA having a novel nucleotide sequence. The present inventors have found that products of interests, such as pregnenolone and the like, can be produced in high concentrations by producing a transformant using DNA encoding the above-described microorganism-derived enzyme protein, and then allowing the enzyme protein-transformed cells, the transformant-treated products and/or the culture to act on a sterol compound used as a raw material. The present invention has been completed based on these findings.

Thus, the present invention provides the following invention.
<1> A protein described in any one of the following (a), (b) and (c):
   (a) a protein consisting of the amino acid sequence shown in SEQ ID NO: 2;
   (b) a protein consisting of an amino acid sequence comprising a deletion, substitution and/or addition of one or several amino acids with respect to the amino acid sequence shown in SEQ ID NO: 2, and having activity of cleaving the bond between positions 20 and 22 of a sterol side chain, wherein the maximum velocity (Vmax) used as a reaction rate parameter of the aforementioned activity is 50 mmol/min/mol or more; and
   (c) a protein consisting of an amino acid sequence having homology of 95% or more to the amino acid sequence shown in SEQ ID NO: 2, and having activity of cleaving the bond between positions 20 and 22 of a sterol side chain, wherein the maximum velocity (Vmax) used as a reaction rate parameter of the aforementioned activity is 50 mmol/min/mol or more.
<2> A sterol side chain cleavage enzyme having the following physicochemical properties:
   (1) action: the enzyme acts on sterol represented by formula (I) as shown below and cleaves the carbon-carbon bond between positions 20 and 22 of a sterol side chain portion by its activity of cleaving the bonds, so as to generate a compound represented by formula (II) as shown below;
   (2) substrate specificity: when microorganisms that produce the enzyme are allowed to react with an aqueous solution containing 100 µg/ml 4-cholesten-3-one or cholesterol at 28°C for 5 hours, the conversion reaction rate from 4-cholesten-3-one to progesterone is 10% or more, and the conversion rate from cholesterol to pregnenolone is 10% or more;
   (3) optimum pH: 7.5 to 8.0;
   (4) optimum temperature for action: 15°C to 20°C;
   (5) thermostability: after preservation at 20°C for 140 hours, 30% or more of enzyme activity is maintained; and
   (6) molecular weight: the putative molecular weight is assumed to be 53 to 54 KDa based on the amino acid sequence, and it is measured to be 50 to 56 KDa by SDS electrophoresis, 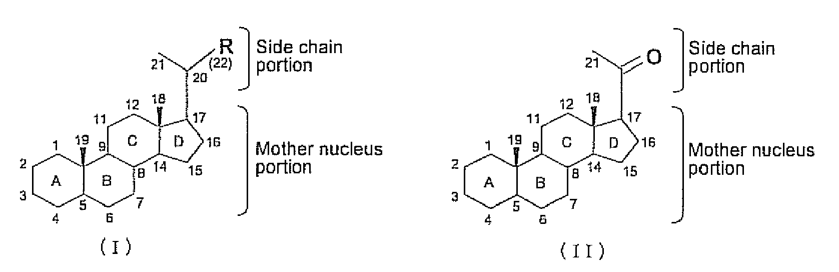 [wherein, in the formula (I),
      the mother nucleus portion has a structure in which it consists of the A, B, C and D rings of a steroid, wherein
      the mother nucleus portion has a carbon-carbon unsaturated bond(s) at zero site or at one or more sites of the positions 1 to 17 of the rings (except for the positions 10 and 13 thereof), and
      carbon(s) at zero site or at one or more sites of the positions 1 to 19 of the rings (except for the positions 10 and 13 thereof) are independently substituted with
      a group represented by the formula -OX
      (namely, a hydroxy group wherein X = H; an acyloxyl group wherein X = COR₁ [wherein R₁ is a hydrogen atom, or an alkyl group, alkenyl group, alkynyl group or aromatic hydrocarbon containing 10 or less carbon atoms]; an O-alkyl group wherein X = R₂ [wherein R₂ is an alkyl group, alkenyl group or alkynyl group containing 10 or less carbon atoms, which may be optionally substituted with an oxygen atom]; a sulfate wherein X = SO₃M [wherein M is a hydrogen atom, an alkaline metal or an alkaline-earth metal]; an O-glycosyl group wherein X is the carbon at position 1 of a sugar; or an epoxy group wherein X is a carbon atom adjacent to said carbon), or
      a keto group represented by the formula =O, and
      in the side chain portion, R is a linear alkyl group, alkenyl group or alkynyl group containing 10 or less carbon atoms, which may have a cyclic portion, or a branched alkyl group, alkenyl group or alkynyl group containing 10 or less carbon atoms, which may have a cyclic portion,
      the carbons at positions 20 and 21, and at zero site or at one or more sites in the R, are independently substituted with
      a group represented by the formula -OY
      (namely, a hydroxy group wherein Y = H; an acyloxyl group wherein Y = COR₃ [wherein R₃ is a hydrogen atom, or an alkyl group, alkenyl group, alkynyl group or aromatic hydrocarbon containing 10 or less carbon atoms]; an O-alkyl group wherein Y = R₄ [wherein R₄ is an alkyl group, alkenyl group or alkynyl group containing 10 or less carbon atoms, which may be optionally substituted with an oxygen atom]; a sulfate wherein Y = SO₃M [wherein M is a hydrogen atom, an alkaline metal or an alkaline-earth metal]; an O-glycosyl group wherein Y is the carbon at position 1 of a sugar; or an epoxy group wherein Y is a carbon atom adjacent to said carbon), or
      a keto group represented by the formula =O], and
      [wherein, in the formula (II),
      the mother nucleus portion has the same definitions as those of the mother nucleus portion of the formula (I), and
      in the side chain portion, the carbon at position 21 is substituted with zero or one group represented by the formula -OY
      (namely, a hydroxy group wherein Y = H; an acyloxyl group wherein Y = COR₃ [wherein R₃ is a hydrogen atom, or an alkyl group, alkenyl group, alkynyl group or aromatic hydrocarbon containing 10 or less carbon atoms]; an O-alkyl group wherein Y = R₄ [wherein R₄ is an alkyl group, alkenyl group or alkynyl group containing 10 or less carbon atoms, which may be optionally substituted with an oxygen atom]; a sulfate wherein Y = SO₃M [wherein M is a hydrogen atom, an alkaline metal or an alkaline-earth metal]; or an O-glycosyl group wherein Y is the carbon at position 1 of a sugar), or
      zero or one keto group represented by the formula=O].
<3> DNA encoding the protein according to <1>.
<4> DNA described in any one of the following (a), (b) and (c):
   (a) DNA having the nucleotide sequence shown in SEQ ID NO: 1;
   (b) DNA having a nucleotide sequence comprising a deletion, substitution and/or addition of one or several nucleotides with respect to the nucleotide sequence shown in SEQ ID NO: 1, and encoding a protein which has activity of cleaving the bond between positions 20 and 22 of a sterol side chain, wherein the maximum velocity (Vmax) used as a reaction rate parameter of the aforementioned activity is 50 mmol/min/mol or more; and
   (c) DNA having a nucleotide sequence capable of hybridizing under stringent conditions with DNA having the nucleotide sequence shown in SEQ ID NO: 1 or a complementary sequence thereof, and encoding a protein which has activity of cleaving the bond between positions 20 and 22 of a sterol side chain, wherein the maximum velocity (Vmax) used as a reaction rate parameter of the aforementioned activity is 50 mmol/min/mol or more.
<5> A fusion protein, wherein the enzyme protein according to <1> or <2>, a ferredoxin protein having electron-transferring activity on the enzyme protein, and a ferredoxin reductase protein having electron-transferring activity on the ferredoxin protein are allowed to bind to one another, so that they can function as a single protein.
<6> A recombinant vector comprising the DNA according to <3> or <4> and an expression control region capable of expressing a protein encoded by the DNA in a host cell.
<7> A transformant obtained by introducing the recombinant vector according to <6> into a host cell.
<8> A method for producing a compound represented by formula (II) as shown below, which comprises allowing a sterol represented by formula (I) as shown below to come into contact with the following (i) or (ii):
   (i) a mixture of the enzyme protein according to <1> or <2>, a ferredoxin protein having electron-transferring activity on the enzyme protein, and a ferredoxin reductase protein having electron-transferring activity on the ferredoxin protein; or
   (ii) the fusion protein according to <5>, 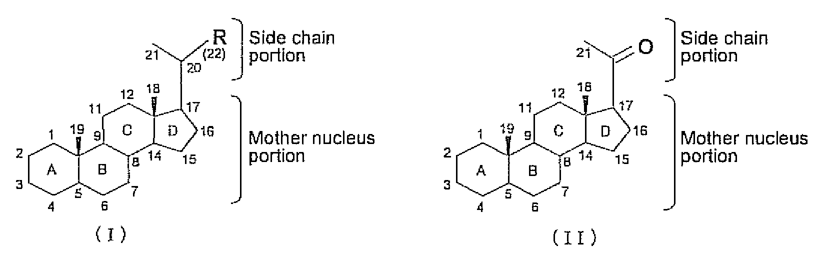 [wherein the definitions of the formula (I) and the formula (II) are the same as those of <1>].
<9> A method for producing a compound represented by formula (II) as shown below, which comprises allowing a sterol represented by formula (I) as shown below to come into contact with any one of the following (a) to (c):
   (a) a mixture of a protein consisting of the amino acid sequence shown in SEQ ID NO: 23, a ferredoxin protein having electron-transferring activity on the aforementioned protein, and a ferredoxin reductase protein that transfers electron to the ferredoxin protein;
   (b) a mixture of: a protein consisting of an amino acid sequence comprising a deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transferring activity on the aforementioned protein; and a ferredoxin reductase protein that transfers electron to the ferredoxin protein; and
   (c) a fusion protein, wherein a protein consisting of the amino acid sequence shown in SEQ ID NO: 23 or a protein consisting of an amino acid sequence comprising a deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transferring activity on the enzyme protein; and a ferredoxin reductase protein having electron-transierring activity on the ferredoxin protein are allowed to bind to one another, so that they can function as a single protein, 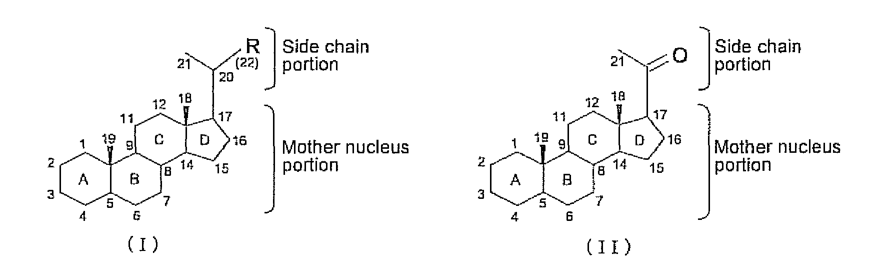 [wherein the definitions of the formula (I) and the formula (II) are the same as those of <1>].
<10> The method according to <8> or <9>, which comprises allowing 3-hydroxysteroid dehydrogenase/isomerase, steroid 17α-hydraxylase, steroid 21-hydroxylase and steroid 11β-hydroxylase to further come into contact with the compound of the formula (II), so as to generate hydrocortisone.
<11> The method according to any one of <8> to <10>, wherein the sterol represented by the above formula (I) is cholesterol, 4-cholesten-3-one, 7-dehydrocholesterol, ergosterol, β-sitosterol, stigmasterol, campesterol, desmosterol, (20S)-20-hydroxycholest-4-en-3-one, (22R)-22-hydroxycholest-4-en-3-one, (20R,22R)-20,22-dihydroxycholest-4-en-3-one, or (20R,22S)-20,22-dihydroxycholest-4-en-3-one.
<12> The method according to any one of <8> to <11>, wherein the compound represented by the above formula (II) is pregnenolone, progesterone, or 7-dehydropregnenolone.
<13> The method according to any one of <8> to <12>, wherein the sterol represented by the above formula (I) is generated by allowing a raw material selected from among glucose, glycerol, methanol, ethanol, sucrose, acetic acid and citric acid, to come into contact with a yeast having ability to assimilate the raw material so as to generate the sterol represented by the above formula (I).
<14> A method for producing hydrocortisone from a raw material selected from among glucose, glycerol, methanol, ethanol, sucrose, acetic acid and citric acid, wherein the method comprises culturing, in a medium containing the aforementioned raw material, a yeast having activity of generating a sterol represented by formula (I) as shown below from the aforementioned raw material, having activity of generating sterol 3beta-hydroxysteroid dehydrogenase and/or 3beta-hydroxysteroid:oxygen oxidoreductase, steroid 17α-hydroxylase, steroid 21-hydroxylase and steroid 11β-hydroxylase, and also having any one of the following characteristics (A) to (E):
   (A) having activity of generating a mixture of the enzyme protein according to <1> or <2>, a ferredoxin protein having electron-transferring activity on the enzyme protein, and a ferredoxin reductase protein having electron-transferring activity on the ferredoxin protein;
   (B) having activity of generating a mixture of a protein consisting of the amino acid sequence shown in SEQ ID NO: 23, a ferredoxin protein having electron-transferring activity on the aforementioned protein, and a ferredoxin reductase protein that transfers electron to the ferredoxin protein;
   (C) having activity of generating a mixture of: a protein consisting of an amino acid sequence comprising deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transferring activity on the aforementioned protein; and a ferredoxin reductase protein that transfers electron to the ferredoxin protein;
   (D) having activity of generating a fusion protein, wherein a protein consisting of the amino acid sequence shown in SEQ ID NO: 23 or a protein consisting of an amino acid sequence comprising a deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transfening activity on the enzyme protein; and a ferredoxin reductase protein having electron-transferring activity on the ferredoxin protein are allowed to bind to one another, so that they can function as a single protein; and
   (E) having activity of generating a fusion protein, wherein the enzyme protein according to <1> or <2>, a ferredoxin protein having electron-transferring activity on the enzyme protein, and a ferredoxin reductase protein having electron-transferring activity on the ferredoxin protein are allowed to bind to one another, so that they can function as a single protein, 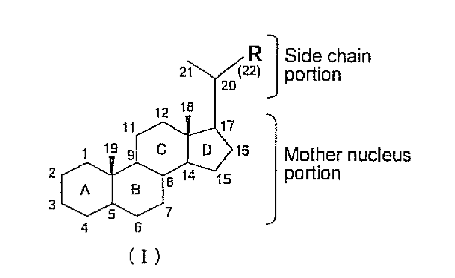 [wherein the definitions of the formula (I) are the same as those of <1>].

### Advantages of the Invention

Using a novel enzyme protein provided by the present invention, which has activity of cleaving the bond between positions 20 and 22 of a sterol compound side chain, it became possible to efficiently produce pregnenolone and the like and hydrocortisone, which are compounds industrially useful as medicaments and pharmaceutical intermediates. In general, when animal-derived P450scc is allowed to express in a microorganism as a host, it is obtained in the form of an insoluble protein, and thus, it is difficult to obtain the protein as an active body. However, when microorganism-derived P450scc provided by the present invention for the first time is allowed to express in a microorganism as a host, it can be obtained in the form of a soluble protein. Thus, since the protein can easily be obtained as an active body, it has high industrial usefulness.

### Best Mode for Carrying Out the Invention

Hereinafter, the embodiments of the present invention will be described in detail.

### (1) Protein of the present invention

According to the present invention, there is provided a protein described in any one of the following (a), (b) and (c) (hereinafter referred to as "CYPSS204A" at times):
(a) a protein consisting of the amino acid sequence shown in SEQ ID NO: 2;
(b) a protein consisting of an amino acid sequence comprising a deletion, substitution and/or addition of one or several amino acids with respect to the amino acid sequence shown in SEQ ID NO: 2, and having activity of cleaving the bond between positions 20 and 22 of a sterol side chain, wherein the maximum velocity (Vmax) used as a reaction rate parameter of the aforementioned activity is 50 mmol/min/mol or more; and
(c) a protein consisting of an amino acid sequence having homology of 95% or more with the amino acid sequence shown in SEQ ID NO: 2, and having activity of cleaving the bond between positions 20 and 22 of a sterol side chain, wherein the maximum velocity (Vmax) used as a reaction rate parameter of the aforementioned activity is 50 mmol/min/mol or more.

Preferably, with regard to the activity of cleaving the bond between positions 20 and 22 of a sterol side chain of the protein of the present invention, the activity decrease rate is 30% or less, when the substrate concentration is increased from 10 µM to 100 µM.

In the present invention, the term "one or several" in the expression "an amino acid sequence comprising a deletion, substitution and/or addition of one or several amino acids" means, for example, approximately 1 to 20, preferably approximately 1 to 10, and more preferably approximately 1 to 5.

The enzyme protein of the present invention also has the following physicochemical properties.

Action: The enzyme acts on sterol represented by formula (I) as shown below and cleaves the carbon-carbon bond between positions 20 and 22 of the sterol side chain portion by its activity of cleaving the bonds, so as to generate a compound represented by formula (II) as shown below. Substrate specificity: When microorganisms that produce the enzyme are allowed to react with an aqueous solution containing 100 µg/ml 4-cholesten-3-one or cholesterol at 28 °C for 5 hours, the conversion reaction rate from 4-cholesten-3-one to progesterone is 10 % or more (preferably 30 % or more, more preferably 50 % or more, further preferably 60 % or more, and particularly preferably 70 % or more), and the conversion reaction rate from cholesterol to pregnenolone is 10 % or more (preferably 30 % or more, more preferably 50 % or more, further preferably 60 % or more, and particularly preferably 65 % or more). 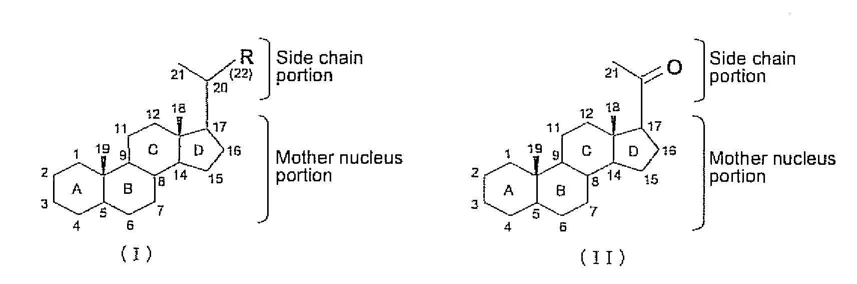 [wherein, in the formula (I), the mother nucleus portion has a structure in which it consists of the A, B, C and D rings of a steroid, wherein the mother nucleus portion has a carbon-carbon unsaturated bond(s) at zero site or at one or more sites of the positions 1 to 17 of the rings (except for the positions 10 and 13 thereof), and carbon(s) at zero site or at one or more sites of the positions 1 to 19 of the rings (except for the positions 10 and 13 thereof) are independently substituted with a group represented by the formula -OX (namely, a hydroxy group wherein X = H; an acyloxyl group wherein X = COR₁ [wherein R₁ is a hydrogen atom, or an alkyl group, alkenyl group, alkynyl group or aromatic hydrocarbon containing 10 or less carbon atoms]; an O-alkyl group wherein X = R₂ [wherein R₂ is an alkyl group, alkenyl group or alkynyl group containing 10 or less carbon atoms, which may be optionally substituted with an oxygen atom]; a sulfate wherein X = SO₃M [wherein M is a hydrogen atom, an alkaline metal or an alkaline-earth metal]; an O-glycosyl group wherein X is the carbon at position 1 of a sugar; or an epoxy group wherein X is a carbon atom adjacent to said carbon), or a keto group represented by the formula =O, and
in the side chain portion, R is a linear alkyl group, alkenyl group or alkynyl group containing 10 or less carbon atoms, which may have a cyclic portion, or a branched alkyl group, alkenyl group or alkynyl group containing 10 or less carbon atoms, which may have a cyclic portion, the carbons at positions 20 and 21, and at zero site or at one or more sites in the R, are independently substituted with a group represented by the formula -OY (namely, a hydroxy group wherein Y = H; an acyloxyl group wherein Y = COR₃ [wherein R₃ is a hydrogen atom, or an alkyl group, alkenyl group, alkynyl group or aromatic hydrocarbon containing 10 or less carbon atoms]; an O-alkyl group wherein Y = R₄ [wherein R₄ is an alkyl group, alkenyl group or alkynyl group containing 10 or less carbon atoms, which may be optionally substituted with an oxygen atom]; a sulfate wherein Y = SO₃M [wherein M is a hydrogen atom, an alkaline metal or an alkaline-earth metal]; an O-glycosyl group wherein Y is the carbon at position 1 of a sugar; or an epoxy group wherein Y is a carbon atom adj acent to said carbon), or a keto group represented by the formula =O], and
[wherein, in the formula (II), the mother nucleus portion has the same definitions as those of the mother nucleus portion of the formula (I), and

in the side chain portion, the carbon at position 21 is substituted with zero or one group represented by the formula -OY (namely, a hydroxy group wherein Y = H; an acyloxyl group wherein Y = COR₃ [wherein R₃ is a hydrogen atom, or an alkyl group, alkenyl group, alkynyl group or aromatic hydrocarbon containing 10 or less carbon atoms]; an O-alkyl group wherein Y = R₄ [wherein R₄ is an alkyl group, alkenyl group or alkynyl group containing 10 or less carbon atoms, which may be optionally substituted with an oxygen atom]; a sulfate wherein Y = SO₃M [wherein M is a hydrogen atom, an alkaline metal or an alkaline-earth metal]; or an O-glycosyl group wherein Y is the carbon at position 1 of a sugar), or zero or one keto group represented by the formula =O].

The sterol of the formula (I) is preferably cholesterol, 4-cholesten-3-one, 7-dehydrocholesterol, ergosterol, β-sitosterol, stigmasterol, campesterol, desmosterol, (20S)-20-hydroxycholest-4-ene-3-one, (22R)-22-hydroxycholest-4-ene-3-one, (20R,22R)-20,22-dihydroxycholest-4-ene-3-one, or (20R,22S)-20,22-dihydroxycholest-4-ene-3-one; and more preferably cholesterol, 4-cholesten-3-one, or 7-dehydrocholesterol.

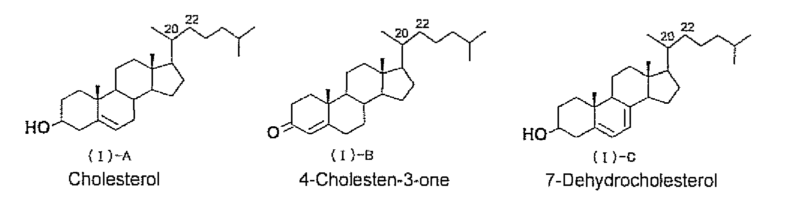

The compound of the formula (II) is preferably pregnenolone, progesterone, or 7-dehydropregnenolone.

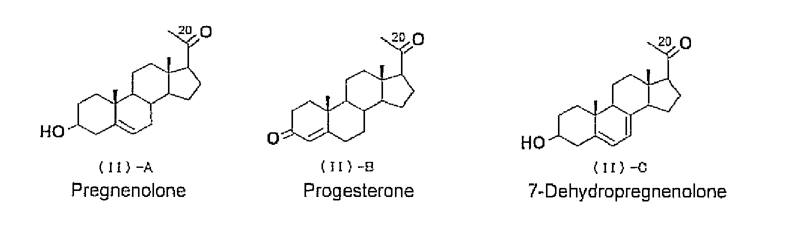

As other properties of the protein of the present invention, the conversion rate from 7-dehydrocholesterol to 7-dehydropregnenolone is 10 % or more, preferably 20 % or more, and more preferably 25 % or more; the conversion rate from ergosterol to 7-dehydropregnenolone is 1 % or more, and preferably 2 % or more; the conversion rate from β-sitosterol to pregnenolone is 10 % or more, preferably 20 % or more, more preferably 30 % or more, and further preferably 40 % or more; and the conversion rate from stigmasterol to pregnenolone is 1 % or more, preferably 2 % or more, and more preferably 5 % or more. Moreover, the conversion rate from campesterol to pregnenolone is 10 % or more, preferably 20 % or more, and more preferably 25 % or more; the conversion rate from desmosterol to pregnenolone is 10 % or more, preferably 30 % or more, more preferably 50 % or more, further preferably 60 % or more, and particularly preferably 65 % or more; the conversion rate from (20S)-20-hydroxycholest-4-en-3-one to progesterone is 10 % or more, preferably 20 % or more, more preferably 30 % or more, and further preferably 40 % or more; the conversion rate from (22R)-22-hydroxycholest-4-en-3-one to progesterone is 10 % or more, preferably 20 % or more, more preferably 30 % or more, and further preferably 40 % or more; the conversion rate from (20R,22R)-20,22-dihydroxycholest-4-en-3-one to progesterone is 10 % or more, preferably 20 % or more, more preferably 30 % or more, and further preferably 35 % or more; and the conversion rate from (20R,22S)-20,22-dihydroxycholest-4-en-3-one to progesterone is 10 % or more, preferably 20 % or more, more preferably 30 % or more, and further preferably 40 % or more. Furthermore, the conversion rate of lanosterol is 0 %.

The enzyme protein of the present invention preferably may further have the following physicochemical properties. Optimum pH: 7.5 to 8.0. Optimum temperature for action: 15 °C to 20 °C. Thermostability: After preservation at 20 °C for 140 hours, 30 % or more of enzyme protein activity is maintained. As for molecular weight, the putative molecular weight is assumed to be 53 to 54 KDa based on the amino acid sequence, and it is measured to be 50 to 56 KDa by SDS electrophoresis. In addition, the maximum velocity (Vmax) used as a reaction rate parameter of the aforementioned activity is 50 mmol/min/mol or more, and the activity decrease rate is 30 % or less when the substrate concentration is increased from 10 µM to 100 µM.

Vmax and Km can be obtained by conducting the conversion of 4-cholesten-3-one at a substrate concentration of 0.1 to 500 µM and then examining the relationship between the substrate concentration and the activity of the enzyme protein. Moreover, the activity decrease rate when the substrate concentration is increased from 10 µM to 100 µM can also be obtained by conducting the conversion of 4-cholesten-3-one at a substrate concentration of 0.1 to 500 µM and then examining the relationship between the substrate concentration and the activity of the enzyme protein. Specifically, there is prepared 1 ml of a reaction solution, which comprises 220 pmol CYP204A1 or 250 pmol CYPSS204A, 96 µg/ml spinach-derived ferredoxin, 0.1 U/ml spinach-derived ferredoxin reductase protein, 3 U/ml glucose dehydrogenase, 60 mM glucose, 2 mM NADH and 2 mM NAD PH. Tris-HCl is then added to the reaction mixture to adjust to pH 7.5. 20 µl of 4-cholesten-3-one used as a substrate, dissolved in DMSO to have a final concentration of 0.1, 0.5, 1, 2, 5, 10, 20, 50, 100 or 500 µM, is added to the reaction mixture. The reaction is initiated by addition of NADH and NADPH, and it can be carried out at 200 rpm at 15 °C for 60 minutes.

The origin of the enzyme protein of the present invention is not particularly limited. It is preferably an enzyme protein derived from microorganisms. Thus, the origin of the present enzyme protein is, for example, *Sphingomanas subterranea,* and most preferably, *Sphingomonas subterranea* NBRC16086. The present enzyme protein may also be derived from *Novosphingobium aromaticivorans* such as *Novosphingobium aromaticivorans* ATCC 700278, as long as it has the above-described amino acid sequence or the above-described properties.

### (2) Method for obtaining enzyme protein of the present invention

The enzyme protein of the present invention can be obtained from microorganisms such as the above-described *Sphingomonas subterranea* or *Novosphingobium aromaticivorans* according to ordinary protein extraction and purification methods. Specific examples of the extraction method include: extraction by cell disintegration, such as chopping with scissors, homogenization, a sonic treatment, osmotic shock procedure, and a freezing and thawing method; extraction using a surfactant; and a combined use thereof. Specific examples of the purification method include salting-out using ammonium sulfate (ammonium sulphate), sodium sulfate or the like, centrifugation, dialysis, ultrafiltration, adsorption chromatography, ion exchange chromatography, hydrophobic chromatography, reverse phase chromatography, gel filtration, gel permeation chromatography, affinity chromatography, electrophoresis, zymography, and a combined use thereof.

Alternatively, the below-mentioned DNA that encodes the protein of the present invention is cloned according to a known method, and the obtained clone is then introduced into a suitable host to allow it to express therein, so that the enzyme protein of the present invention can be obtained. For example, based on the information of the nucleotide sequence of a gene encoding the enzyme protein of the present invention as described in the specification, a probe or a primer specific to the gene of the enzyme protein of the present invention is designed. Using the probe or primer, DNA encoding the enzyme protein of the present invention is isolated or amplified from the DNA library (e.g. a genomic DNA library, a cDNA library, etc.) of microorganisms such as *Sphingomonas subterranea* or *Novosphingobium aromaticivorans.* The obtained DNA is introduced into a vector according to a gene recombinant technique, and the vector is then introduced into a host cell, so that it can be expressed therein, thereby obtaining the enzyme protein of the present invention.

### (3) DNA encoding protein of the present invention, recombinant vector, and transformant

According to the present invention, there is provided DNA encoding the above-described protein of the present invention. A specific example of the DNA of the present invention is DNA described in any one of the following (a), (b) and (c):
(a) DNA having the nucleotide sequence shown in SEQ ID NO: 1;
(b) DNA having a nucleotide sequence comprising a deletion, substitution and/or addition of one or several nucleotides with respect to the nucleotide sequence shown in SEQ ID NO: 1, and encoding a protein which has activity of cleaving the bond between positions 20 and 22 of a sterol side chain, wherein the maximum velocity (Vmax) used as a reaction rate parameter of the aforementioned activity is 50 mmol/min/mol or more; and
(c) DNA having a nucleotide sequence capable of hybridizing under stringent conditions with DNA having the nucleotide sequence shown in SEQ ID NO: 1 or a complementary sequence thereof, and encoding a protein which has activity of cleaving the bond between positions 20 and 22 of a sterol side chain, wherein the maximum velocity (Vmax) used as a reaction rate parameter of the aforementioned activity is 50 mmol/min/mol or more.

A gene encoding the enzyme protein of the present invention can also be isolated from the a DNA library of microorganisms such as *Sphingomonas subterranea* and *Novosphingobium aromaticivorans* by performing hybridization using, as a probe, an oligonucleotide produced based on the nucleotide sequence of *Sphingomonas subterranea, Novosphingobium aromaticivorans* or the like, or by performing PCR using the oligonucleotide produced based on the aforementioned sequence as a primer. Moreover, the gene of the enzyme protein of the present invention may be not only a gene isolated from naturally-existing microorganisms, but also a gene that is synthesized by changing a codon such that the present enzyme protein can be appropriately expressed without the change of the amino acid sequence in a host cell for expressing the enzyme protein.

In the present invention, the term "one or several" in the expression "a nucleotide sequence comprising a deletion, substitution and/or addition of one or several nucleotides" means, for example, approximately 1 to 30, preferably approximately 1 to 20, more preferably approximately 1 to 10, and further preferably approximately 1 to 5. In the present invention, the expression "a nucleotide sequence capable of hybridizing under stringent conditions with ...'' means DNA and the like comprising a nucleotide sequence having homology on BLAST analysis of 90 % or more, and more preferably 95 % or more to DNA having the nucleotide sequence shown in SEQ ID NO: 1 or a complementary sequence thereof. Moreover, the "hybridization that is carried out under stringent condition" can be carried out by a method, which comprises carrying out a reaction in an ordinary hybridization buffer at a temperature of 40 °C to 70 °C, and preferably 60 °C to 65 °C, and then washing the reaction product in a washing solution having a salt concentration of 15 mM to 300 mM, and preferably 15 mM to 60 mM.

A gene having a nucleotide sequence comprising a deletion, substitution and/or addition of one or several nucleotides with respect to the nucleotide sequence shown in SEQ ID NO: 1 can be produced by ordinary mutation operations such as a method using a mutation inducer or site-directed mutagenesis. These mutation operations can be easily carried out using commercially available kits such as Site-Directed Mutagenesis Kit (Takara Shuzo Co., Ltd.) or QuickChange Site-Directed Mutagenesis Kit (manufactured by STRATAGENE).

According to the present invention, there is further provided a recombinant vector having the DNA of the present invention. The recombinant vector means a recombinant vector comprising the DNA of the present invention and an expression control region capable of expressing a protein encoded by the DNA in a host cell. Specifically, it is a vector generally comprising the DNA of the present invention and a promoter suitable for a host microorganism, wherein the 5'-terminal side of the coding region of the DNA of the present invention is ligated downstream of the promoter. The type of such a vector is not particularly limited, as long as it is capable of replicating in a host microorganism. Examples of the vector include a plasmid vector, a shuttle vector, and a phage vector. Specific examples of the plasmid vector include pBR322, pUC18, pHSG298, pUC118, pSTV28, pTWV228, pHY300PLK (manufactured by Takara Shuzo Co., Ltd., etc.), pKK223-3, and pPL-lambda inducible expression vector (manufactured by Pharmacia, etc.). Specific examples of shuttle vectors of *Escherichia coli*-coryneform group of bacteria include pCRY30 (JP Patent Publication (Kokai) No. 3-210184 A (1991)), pCRY21 (JP Patent Publication (Kokai) No. 2276575 A), pCRY2KE, pCRY2KX, pCRY31, pCRY3KE and pCRY3KX, pCRY2 and pCRY3 (JP Patent Publication (Kokai) No. 1-191686 A (1989)), pAM330 (JP Patent Publication (Kokai) No. 58-67679 A (1983)), pHM1519 (JP Patent Publication (Kokai) No. 58-77895 A (1983)), pAJ655, pAJ611 and pAJ1844 (JP Patent Publication (Kokai) No. 58-192900 A (1983)), pCG1 (JP Patent Publication (Kokai) No. 57-134500 A (1982)), pCG2 (JP Patent Publication (Kokai) No. 58-35197 A (1983)), pCG4 and pCG11 (JP Patent Publication (Kokai) No. 57-183799 A (1982)), and also their derivatives. In addition, an example of the phage vector is a λFixII vector (manufactured by STRATAGENE, etc.).

On the other hand, when Eumycetes such as yeast are used, there can be applied a method of using a vector by which multiple copies are introduced into the chromosome, among chromosome-introduced-type plasmids (Sakai, A. et al., (1991) Biotechnology (NY) 9, 1382-1385), and the use of multicopy plasmids such as a 2-µm plasmid-derived pESC vector (manufactured by STRATAGENE) or a pAUR vector (manufactured by Takara Bio INC.), among non-chromosome-introduced-type plasmids.

As a promoter for the expression of the DNA encoding the enzyme protein of the present invention in yeast used as a host, there can be used a constant expression promoter for the expression of PGK1 gene encoding phoshoglycerate kinase, ADH1 gene encoding alcohol dehydrogenase, GAP1 gene encoding general amino acid permease, etc. Moreover, an inducible promoter such as GAL1/GAL10 (Johnston, M. et al., (1984) Mol. Cell. Biol. 4(8), 1440-1448) can also be used. When these promoters are used, expression is induced by adding galactose in the absence of glucose.

As a promoter for the expression of the DNA encoding the enzyme protein of the present invention, a promoter possessed by a host microorganism can be generally used. However, the type of a promoter is not limited thereto, and all types of promoters may be used, as long as they have nucleotide sequences for initiating the transcription of the gene of the enzyme protein of the present invention. Specific examples of such a promoter include a lactose operon promoter, a tryptophan operon promoter, a λ-phage-derived PL promoter, and a tryptophan-lactose hybrid (tac) promoter (H. A. Bose et al., Proc. Natl. Acad. Sci. U.S.A., Vol. 80, p. 21 (1983)). Among these promoters, an inducible promoter can be used for the purpose of improving expression efficiency. For example, in the case of the above-described lactose operon promoter, gene expression can be induced by adding lactose or isopropyl-β-D-thiogalactoside (IPTG).

According to the present invention, there is further provided a transformant formed by introducing the DNA of the present invention or a recombinant vector into a host cell. The type of such a host cell, into which the DNA of the present invention or a recombinant vector is to be introduced, is not particularly limited. Suitable examples of such a host include bacteria of genus Escherichia, such as *Escherichia coli,* bacteria of genus Actinomycetes, bacteria of genus Bacillus, bacteria of genus Serratia, bacteria of genus Pseudomonas, bacteria of genus Corynebacterium, bacteria of genus Brevibacterium, bacteria of genus Rhodococcus, bacteria of genus Lactobacillus, bacteria of genus Streptomyces, bacteria of genus Thermus, bacteria of genus Streptococcus, yeast of genus Saccharomyces, yeast of genus Pichia, yeast of genus Kluyveromyces, yeast of genus Candida, yeast of genus Schizosaccharomyces, yeast of genus Debaryomyces, yeast of genus Yarrowia, yeast of genus Cryptococcus, yeast of genus Xanthophyllomyces, mold of genus Aspergillus, mold of genus Martierella, mold of genus Fusarium, genus Schizochytrium, and genus Thraustochytriurn. Preferred host cells include *Escherichia coli,* Actinomycetes, bacteria of genus Pseudomonas, and yeast of genus Saccharomyces.

Specifically, there can be used *Escherichia coli, Bacillus subtilis, Bacillus brevis, Bacillus stearothermophilus, Serratia marcescens, Pseudomonas putida, Pseudomonas aeruginosa, Corynebacterium glutamicum, Brevibacterium flavum, Brevibacterium lactofermentum, Rhodococcus erythropolis, Thermus thermophilus, Streptococcus lactis, Lactobacillus casei, Streptomyces lividans, Saccharomyces cerevisiae, Saccharomyces bayanus, Pichia pastoris, Kluyveromyces lactis, Candida utilis, Candida glabrata, Schizosaccharomyces pombe, Debaryomyces hansenii, Yarrowia lypolitica, Cryptococcus curvatus, Xanthophyllomyces dendrorhous, Aspergillus nigar, Aspergillus oryzae, Mortierella ramanniana, Mortierella bainieri, Mortierella alpina, Cunninghamella elegans, Fusarium fujikuroi, Schizochytrium limacium, Thraustochytrium aureum.,* etc.

As a method of introducing a gene into the above-described host microorganisms, transformation methods such as the competent cell method [Journal of Molecular Biology, Vol. 53, p. 159 (1970)], the lithium acetate method [Ito, H. et al. J. Bacteriol., Vol. 153, p. 163 (1983)], a spheroplast method [Hinnen, A., et al. Proc. Natl. Acad. Sci. USA, Vol. 75, p. 1929 (1978)] or the pulse wave electrification method [J. Indust. Microbiol., Vol. 5, p. 159 (1990)], the transduction method using phage [E. Ohtsubo, Genetics, Vol. 64, p. 189 (1970)], the conjugal transfer method [J. G. C. Ottow, Ann. Rev. Microbiol., Vol. 29, p. 80 (1975)], the cell fusion method [M. H. Gabor, J. Bacteriol., Vol. 137, p. 1346 (1979)], etc. can be applied. From these methods, a method suitable for host microorganisms may be selected, as appropriate.

In addition to the above-described gene expression method using an expression vector, the gene may also be expressed by a homologous recombination technique whereby the DNA encoding the enzyme protein of the present invention ligated to a promoter is directly introduced into the chromosome of a host microorganism, or by a technique of introducing the gene using transposon, or the insertion sequence or the like. Accordingly, the transformant of the present invention is sufficient as long as the enzyme protein of the present invention is expressed therein. The method of introducing the gene into a host is not limited.

### (4) Production of enzyme protein of the present invention using transformant of the present invention

According to the present invention, a transformant obtained as described in (3) above may be cultured, and the enzyme protein of the present invention may be then collected from the culture.

The transformant can be cultured in an ordinary nutritive medium containing a carbon source, a nitrogen source, inorganic salts, various types of vitamins, etc. Examples of the carbon source used herein include: sugars such as glucose, sucrose, fructose or maltose; alcohols such as ethanol or methanol; organic acids such as citric acid, malic acid, succinic acid, maleic acid or fumaric acid; and blackstrap molasses. As a nitrogen source, ammonia, ammonium sulfate, ammonium chloride, ammonium nitrate, urea and the like are used singly or in combination. Examples of the inorganic salts used herein include potassium monohydrogen phosphate, potassium dihydrogen phosphate, and magnesium sulfate. Other than these substances, nutritive substances such as peptone, meat extract, yeast extract, corn steep liquor, casamino acid, and various types of vitamins such as biotin, may be added to the medium.

The culture is generally carried out under aerobic conditions involving aeration stirring or shaking. The culture temperature is not particularly limited, as long as it allows host microorganisms to grow. In addition, the pH applied during the culture is not particularly limited, either, as long as it allows host microorganisms to grow. During the culture, the pH value can be adjusted by addition of acid or alkali.

An enzyme protein can be collected from the culture according to a known collection method, using the activity of the enzyme protein as an indicator. It is not always necessary to purify the enzyme protein to a homogeneous state. The enzyme protein may be purified up to a purification degree that depends on intended use.

As a roughly purified fraction or a purified enzyme protein used in the present invention, a cell mass separated from culture media obtained as a result of the culture of a transformant; a crushed product obtained by crushing culture media or cell mass by means such as ultrasonic wave or friction under pressure; an extract containing the enzyme protein of the present invention, which is obtained by extracting the crushed product with water or the like; a crude preparation of the enzyme protein of the present invention, which is obtained by further performing a treatment such as ammonium sulfate fractionation or column chromatography on the above-described extract; or a purified enzyme protein preparation, may also be used. Furthermore, a product obtained by immobilizing the above-described cell mass, crushed product, extract, roughly purified fraction or purified enzyme protein on a carrier may also be used.

The above-described cell mass, cell mass crushed product, extract, or purified enzyme protein can be immobilized on a carrier according to a known ordinary method of immobilizing such cell mass or the like on suitable carrier such as acrylamide monomer, alginic acid or carrageenan. For example, when the cell mass is immobilized on the carrier, the cell mass, which has just been recovered from the culture or has been washed with suitable buffer such as approximately 0.02 to 0.2 M phosphate buffer (pH 6 to 10), can be used.

### (5) Method for producing pregnenolone, progesterone and 7-dehydropregnenolone using enzyme protein of the present invention and the like

According to the present invention, the sterol represented by the above formula (I) is allowed to come into contact with (i) a mixture of the above- described enzyme protein (CYPSS204A) of the present invention, the ferredoxin protein having electron-transferring activity on the enzyme protein, and the ferredoxin reductase protein having electron-transferring activity on the ferredoxin protein, or (ii) a fusion protein of the enzyme protein of the present invention, the ferredoxin protein having electron-transferring activity on the enzyme protein, and the ferredoxin reductase protein having electron-transferring activity on the ferredoxin protein, so as to produce the compound represented by the formula (II).

In the present invention, other than the above-described proteins, the sterol of the formula (I) is allowed to come into contact with (a) mixture of the protein (CYP204 A1) consisting of the amino acid sequence shown in SEQ ID NO: 23, the ferredoxin protein having electron-transferring activity on the aforementioned protein, and the ferredoxin reductase protein that transfers electron to the ferredoxin protein, (b) a mixture of the protein consisting of an amino acid sequence comprising deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; the ferredoxin protein having electron-transferring activity on the aforementioned protein; and the ferredoxin reductase protein that transfers electron to the ferredoxin protein, or (c) a fusion protein, wherein a protein consisting of the amino acid sequence shown in SEQ ID NO: 23 or the protein consisting of an amino acid sequence comprising deletion, substitution and/or addition of one or several amino acids with respect to the amino acid sequence shown in SEQ JD NO: 23, and having activity of cleaving the bond between positions 20 and 22 of the sterol side chain, wherein the maximum velocity (Vmax) used as a reaction rate parameter of the aforementioned activity is 40 mmol/min/mol or more; the ferredoxin protein having electron-transferring activity on the enzyme protein; and the ferredoxin reductase protein having electron-transferring activity on the ferredoxin protein are allowed to bind to one another, so that they can function as a single protein, thereby producing the compound represented by the formula (II).

Herein, the ferredoxin protein having electron-transferring activity and the ferredoxin reductase protein having electron-transferring activity on the ferredoxin protein may be a single protein having the activities of the two above proteins. For example, there can be used fusion protein, which is artificially produced by ligating the P450 reductase domain of the P450-BM3 gene of *Bacillus megaterium* to the enzyme protein (CYP204A1) (Helvig, C. and Capdevila, J. H., Biochemistry, (2000) 39, 5196-5205). Moreover, the above-described protein may be allowed to come into contact with the sterol represented by the above formula (I) by adding the enzyme protein to the sterol, or by allowing microorganisms that produce the above-described protein or a treated product thereof, or the above described transformant or a treated product thereof, to come into contact with the sterol.

With regard to ferredoxin and ferredoxin reductase, the ferredoxin reductase is selected from, for example, the following: spinach-derived ferredoxin reductase, *Pseudomonas putida*-derived putida redoxin reductase, animal-derived adrenodoxin reductase, *Sphingomonas subterranea*-derived ferredoxin reductase, *Novosphingobium aromaticivorans*-derived ferredoxin reductase, *Escherichia* coli-derived flavodoxin reductase or ferredoxin reductase, *Saccharomyces cerevisiae*-derived ferredoxin reductase, and other ferredoxin reductases. The ferredoxin is selected from, for example, the following: spinach-derived ferredoxin, *Pseudomonas putida*-derived putida redoxin, animal-derived adrenodoxin. *Sphingomonas subterranea*-derived ferredoxin, *Novosphingobium aromaticivorans*-derived ferredoxin, *Escherichia coli*-derived flavotoxin or ferredoxin, *Saccharomyces cerevisiae*-derived ferredoxin, and other ferredoxin-type proteins.

The reaction method is not particularly limited. The sterol represented by the formula (I) used as a substrate is added to the liquid containing the enzyme protein of the present invention (CYPSS204A) and CYP204A1, or microorganisms that produce the aforementioned enzyme protein, etc., and the mixed solution is then reacted at an appropriate temperature such as approximately 10°C to 40°C. By this reaction, the compound represented by the formula (II), such as pregnenolone, progesterone or 7-dehydropregnenolone, can be produced. Alternatively, even in a case in which microorganisms that produce the enzyme protein of the present invention intrinsically generate the sterol represented by the formula (I), they are reacted so as to produce the compound represented by the formula (II).

A method of fractionating the compound of interest represented by the formula (II) from the reaction mixture is not particularly limited. A separation or purification method known to a person skilled in the art can be applied. The compound of the formula (II) can be fractionated, for example, by solvent extraction, crystallization, resin adsorption, column chromatography, and the like, but the method is not limited thereto.

### (6) Method for producing hydrocortisone and derivative thereof

In the method of producing the compound represented by the above formula (II), steroid 17α-hydroxylase protein, steroid 21-hydroxylase protein, steroid 113-hydroxylase protein, 3beta-hydroxysteroid dehydrogenase and/or 3beta-hydroxysteroid:oxygen oxidoreductase protein and sterol Δ7-reductase protein are allowed to further come into contact with the compound of the formula (II), so that hydrocortisone can be produced.

The steroid 17α-hydroxylase protein, the steroid 21-hydroxylase protein, the steroid 11β-hydroxylase protein, the 3beta-hydroxysteroid dehydrogenase and/or 3beta-hydroxysteroid:oxygen oxidoreductase protein and the sterol Δ7-reductase protein may be allowed to come into contact with the compound represented by the above formula (II) or a product converted from the compound of the formula (II) as a result of the enzyme reaction of any one of the aforementioned proteins, by adding the enzyme proteins to the compound, or by allowing microorganisms that produce the protein or a treated product thereof, or the above described transformant or a treated product thereof, to come into contact with the compound. The steroid 17α-hydroxylase protein, the steroid 21-hydroxylase protein, the steroid 11β-hydroxylase protein and the 3beta-hydroxysteroid dehydrogenase and/or 3beta-hydroxysteroid:oxygen oxidoreductase protein, which are used herein, may be those described in Molecular and Cellular Endocrinology 1990, 73, 73-80, for example. As a sterol Δ7-reductase protein, a protein derived *from Arabidopsis thaliana,* described in Journal of Biological Chemistry 1996, 271. 10866-10873, or a protein derived from mold of genus Mortierella, described in Applied and Environmental Microbiology 2007, 73, 1736-1741, is used. As a steroid 17α-hydroxylase protein, cytochrome P450cl7 derived from cattle, mouse, rat or human being is preferable, for example. Moreover, as a steroid 21-hydroxylase protein, cytochrome P450c21 derived from cattle, mouse, rat or human being is preferable. As a steroid 11β-hydroxylase protein, cytochrome P450c11 derived from cattle, mouse, rat or human being, *Curvularia lunata*-derived P-4501un, and the like are preferable. Furthermore, as a 3beta-hydroxysteroid dehydrogenase and/or 3beta-hydroxysteroid:oxygen oxidoreductase protein, 3β-hydroxysteroid dehydrogenase (3βHSD) derived from cattle, mouse, rat or human being, and cholesterol oxidase derived from bacteria of genus Streptomyces are preferable. As a sterol Δ7-reductase protein, *Arabidopsis thaliana-*deraved NADPH-sterol Δ7-reductase and *Mortierellla alpina-*derived sterol Δ7-reductase (MoDELTA7SR) are preferable.

Furthermore, the method for producing hydrocortisone of the present invention preferably includes a method for producing hydrocortisone from a raw material selected from among glucose, sucrose, glycerol, methanol, ethanol, citric acid and acetic acid, wherein the method comprises culturing, in a medium containing the aforementioned raw material, a yeast or mold having activity of generating the sterol represented by the above formula (I) from the aforementioned raw material_{;} also having activity of generating sterol Δ7-reductase, 3beta-hydroxysteroid dehydrogenase and/or 3beta-hydroxysteroid:oxygen oxidoreductase, steroid 17α-hydroxylase, steroid 21-hydroxylase and steroid 11β-hydroxylase, and also having any one of the following characteristics (A) to (E):
(A) having activity of generating a mixture of the enzyme protein according to claim 1 or 2, the ferredoxin protein having electron-transferring activity on the enzyme protein, and the ferredoxin reductase protein having electron-transferring activity on the ferredoxin protein;
(B) having activity of generating a fusion protein, wherein the enzyme protein according to claim 1 or 2, the ferredoxin protein having electron-transferring activity on the enzyme protein, and the ferredoxin reductase protein having electron-transferring activity on the ferredoxin protein are allowed to bind to one another, so that they can function as a single protein;
(C) having activity of generating a mixture of the protein consisting of the amino acid sequence shown in SEQ ID NO: 23, the ferredoxin protein having electron-transferring activity on the aforementioned protein, and the ferredoxin reductase protein that transfers electron to the ferredoxin protein;
(D) having activity of generating a mixture of: the protein consisting of an amino acid sequence comprising deletion, substitution and/or addition of one or several amino acids with respect to the amino acid sequence shown in SEQ ID NO: 23, and having activity of cleaving the bond between positions 20 and 22 of the sterol side chain, wherein the maximum velocity (Vmax) used as a reaction rate parameter of the aforementioned activity is 40 mmol/min/mol or more; the ferredoxin protein having electron-transferring activity on the aforementioned protein; and the ferredoxin reductase protein that transfers electron to the ferredoxin protein; and
(E) having activity of generating fusion protein, wherein the protein consisting of the amino acid sequence shown in SEQ ID NO: 23 or the protein consisting of an amino acid sequence comprising deletion, substitution and/or addition of one or several amino acids with respect to the amino acid sequence shown in SEQ ID NO: 23, and having activity of cleaving the bond between positions 20 and 22 of the sterol side chain, wherein the maximum velocity (Vmax) used as a reaction rate parameter of the aforementioned activity is 40 mmol/min/mol or more; the ferredoxin protein having electron-transferring activity on the enzyme protein; and the ferredoxin reductase protein having electron-transferring activity on the ferredoxin protein are allowed to bind to one another, so that they can function as a single protein.

In the present invention, with regard to the description "having activity of generating the protein," there is preferably used a method, which involves the insertion of DNA encoding the protein into the above-described expression vector and the use of a transformed yeast or a transformed mold obtained by transforming the above-described yeast or mold.

Examples of the yeast or mold that is preferably used herein include yeast of genus Saccharomyces, yeast of genus Pichia, yeast of genus Schizosaccharomyces, yeast of genus Yarrowia, mold of genus Aspergillus, and mold of genus Mortierella. A more preferred example is yeast of genus Saccharomyces. Specifically, it is *Saccharomyces cerevisiae.* More specific examples of the *Saccharomyces cerevisiae* include Saccharomyces cerevisiae (JP2004-528827 A1) strain, FY1679-18b strain (JP2004-528827 A1), KA311A strain (FERM:P-19053), YPH499 strain (ATCC#204679), and YPH500 strain (ATCC#204680). DNA encoding each enzyme protein is preferably inserted into an expression vector such as pESC-LEU (STRATAGENE) or the chromosome of the yeast. In addition, these yeasts or molds preferably have any one or all of the following properties (1) to (6): (1) the site between positions 22 and 23 of intrinsic sterol cannot be unsaturated by modifying the DNA sequence of the gene encoding a sterol-22 desaturase protein or a region that controls the expression thereof; (2) the expression of an NADP-cytochrome P450 reductase protein is reinforced by introducing one or more copies of gene encoding the present protein and an expression control region thereof into host cells; (3) intrinsic sterol cannot be esterified by modifying the DNA sequence of a gene encoding sterol esterification enzyme protein or a region that controls the expression thereof; (4) the expression of sterol ester hydrolase is reinforced by introducing one or more copies of gene encoding the present protein and an expression control region thereof into host cells; (5) by introducing gene encoding each of ferredoxin-type protein that transfers electron to steroid 17α-hydroxylase, steroid 21-hydroxylase and steroid 11β-hydroxylase, and ferredoxin reductase protein having electron-transferring activity on the aforementioned protein, and an expression control region thereof into host cells, the expression of the aforementioned proteins is reinforced; and (6) as a result of modification of the DNA sequence of the gene encoding a sterol-24 methyl transferase protein or a region that controls the expression thereof, no methyl groups are present in the position 24 of intrinsic sterol.

A method of fractionating hydrocortisone from the reaction mixture is not particularly limited, and a separation or purification method known to a person skilled in the art can be applied. Hydrocortisone can be fractionated, for example, by solvent extraction, crystallization, resin adsorption, column chromatography, and the like, but the method is not limited thereto.

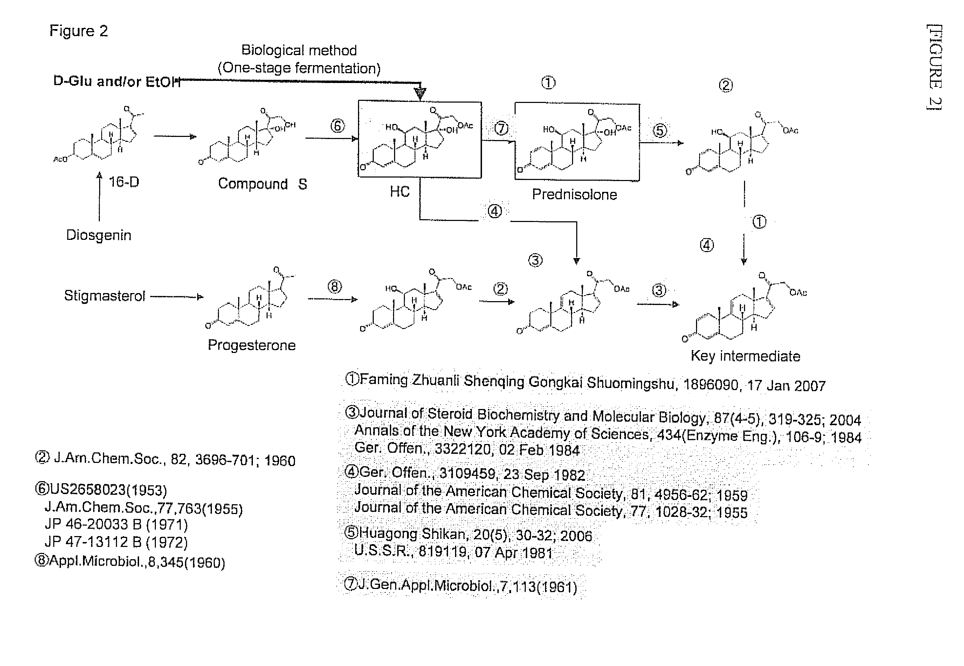

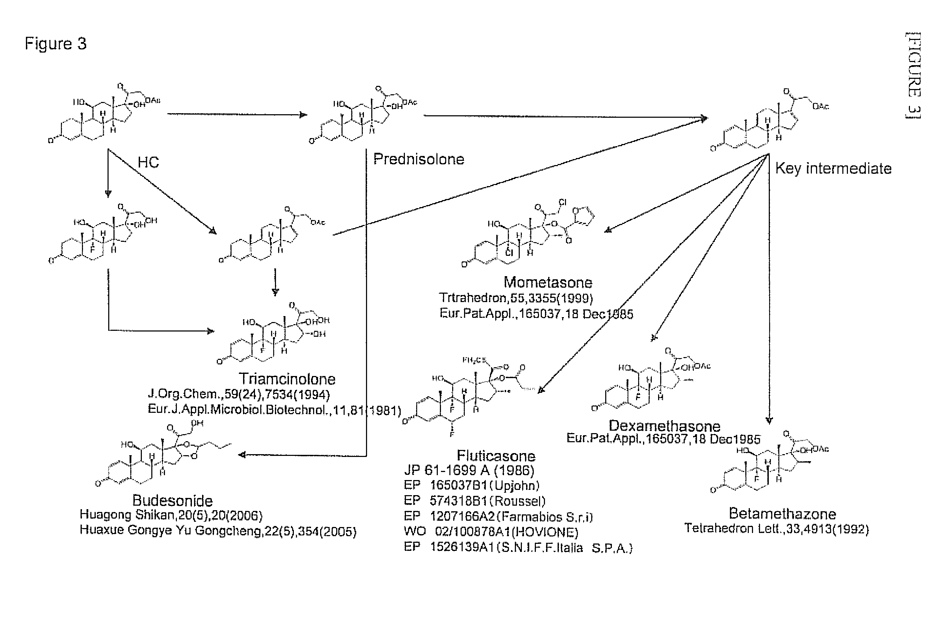

The derivatives shown in Figures 2 and 3 can be produced from the above-produced hydrocortisone according to a known method. Moreover, the thus produced hydrocortisone and derivatives thereof are screened in terms of activity or safety as medicaments by *in vitro* or *in* viva pharmacological or physiological tests according to know methods, and as a result, they can be used as therapeutic agents for diseases. In this case, a method of producing a pharmaceutical composition comprising, as an active ingredient, the compound obtained in the above-described process is also included in the present invention.

Specifically, the compound may be used alone, but it may also be mixed with a pharmaceutically acceptable carrier and may be then used as a pharmaceutical composition. The thus obtained pharmaceutical compound may be used alone, but it may also be mixed with a pharmaceutically acceptable carrier and may be then used as a pharmaceutical composition. The ratio of the active ingredient to the carrier can be fluctuated from 0.01 % to 90% by weight.

The pharmaceutical composition obtained in the present invention may be orally administered in a dosage form such as a tablet coated with sugar as necessary, a capsule, a soft capsule, a powder agent, a granule agent, a fine grain agent, a syrup, an emulsion, a suspension or a liquid agent. Alternatively, the present pharmaceutical composition may be processed into an aseptic solution with a pharmaceutically acceptable liquid or an injection such as a suspension, and it may be administered, for example, via intravenous administration, intramuscular administration, local administration, or subcutaneous administration. It is also possible to administer the present pharmaceutical composition via intramucosal administration in the form of a suppository, a sublingual tablet, a transnasal agent, a transpulmonary agent and the like, or in the form of external preparations such as an ointment a patch and the like. When a composition is prepared for use in oral or parenteral administration, it can be mixed with an organic or inorganic, solid or liquid carrier or diluent, in a unit capacity and a form commonly applied together therewith. The amount of an active ingredient in such a preparation is adjusted, such that an appropriate capacity can be obtained in the designated range.

Examples of an excipient used in the production of a solid preparation for use in oral and transmucosal administration include lactose, sucrose, starch, talc, cellulose, dextrin, kaoline, and calcium carbonate. A liquid preparation for use in oral administration, namely, a syrup, a suspension, a liquid agent or the like, comprises a commonly used inactive diluent, such as water or vegetable oil. This preparation may also comprise auxiliary agents such as a wetting agent, a suspension adjuvant, a sweetener, a flavor, a coloring agent, a preservative and a stabilizer, as well as the aforementioned inactive diluent. Examples of a solvent or a suspending agent used in the production of an injection, a transmucosal agent, etc., include water, propylene glycol, polyethylene glycol, benzyl alcohol, ethyl oleate, and lecithin. Examples of a base used in a suppository include cacao butter, emulsified cacao butter, laurin butter, and Witepsol. In the production of external agents, alcohol, fatty acid esters, propylene glycol, etc. are used as solubilizers or solubilizing agents: a carboxy vinyl polymer, polysaccharide, etc. are used as thickeners; and a surfactant, etc. are used as emulsifiers.

### Examples

General experimental methods applied in the following examples were carried out in accordance with the experimental manual of Sambrook et al. (Sambrook, J., and Russel, D. W.: Molecular cloning: a laboratory manual. 3rd edition. Cold Spring Harbor Laboratory Press, Cold Spring Harbor, New York, 2001).

### Example 1: Obtainment of DNA encoding Sphingomonas subterranea NBRC16086-derived P450scc (CYPSS204A) and ferredoxin

*sphingomonas subterranea* NBRC 16086 (Biological Resource Center, Biotechnology Field, Notional Institute of Technology and Evaluation, Incorporated Administrative Agency) was inoculated into an L medium (1 % tryptone, 0.5 % yeast extract, 0.5 % NaCI, 0.1 % glucose, pH 7.2), and it was then cultured at 30 °C overnight. Thereafter, genomic DNA was extracted from the obtained cell mass. The genomic DNA was extracted using a DNA extraction kit ISOPLANT (manufactured by Wake Pure Chemical Industries, Ltd.) A portion of DNA encoding CYPSS (hereinafter referred to as a "CYPSS204A gene" at times) was amplified from the genomic DNA by a polymerase chain reaction (PCR) using a primer CYP-1F' (SEQ ID NO: 5) and a primer CYP-2R (SEQ ID NO: 6) and using LA Taq polymerase (manufactured by Takara Bio INC.). The temperature conditions applied during the PCR were 94 °C/3 min, 30 cycles of (94 °C/30 sec, 55 °C/30 sec, and 72 °C/90sec), and 72 °C/5 min.

The nucleotide sequence of a 1.0-kb gene fragment amplified as a result of the above-described reaction was determined. Based on the obtained nucleotide sequence information, inverse PCR was carried out, and the entire nucleotide sequence (SEQ ID NO: 3) of a 1.8-kb DNA region comprising the above-described CYP204A1 gene and a ferredoxin gene located downstream thereof was determined. The nucleotide sequence of the CYPSS204A gene (nucleotides 1 to 1419 of SEQ ID NO: 3) is shown in SEQ ID NO: 1, and the amino acid sequence of CYPYP204A1 is shown in SEQ ID NO: 2. In the nucleotide sequence shown in SEQ ID NO: 1, nucleotides 1430 to 1762 correspond to the ferredoxin.

### Example 2: Production of Escherichia coli expression strain BP215 that expresses Sphingomonas subterranea NBRC 16086-deprived P450scc (CYPSS204A)

A DNA region comprising CYPSS204A and a ferredoxin gene located downstream thereof was amplified from the *Sphingomonas subterranea* NBRC 16086 genomic DNA obtained in Example 1 by PCR using a primer CYP-3F (SEQ ID NO: 7) and a primer CYP-4R (SEQ ID NO: 8) and also using KOD plus polymerase (manufactured by TOYOBO Co., Ltd.). The temperature conditions applied during the PCR were 95 °C/5 min, 30 cycles of (98 °C/30 sec, 60 °C/30 sec, and 68 °C/2 min), and 68 °C/10 min.

The amplified 1.8-kb DNA fragment was purified using QIAquick PCR purification Kit (manufactured by QIAGEN), and the resultant was then digested with Nde I and Spe I. Thereafter, using T4 DNA ligase, the digest was ligated to an *Escherichia coli* expression vector pT7NS-camAB (International Publication WO2003/087381, pamphlet), and *Escherichia coli* DH5 alpha was then transformed with the expression vector, so as to construct a plasmid pCYPSS-camAB. *Escherichia coli* BL21(DE3) was transformed with this plasmid, and the obtained cell strain was named as BP215.

### Example 3: Obtainment of DNA encoding P450scc (CYP204A1) from Novosphingobium aromaticivorans ATCC 700278 and production of Escherichia coli expression strain BP172

*Novosphingobium aromaticivorans* (ATCC 700278) was inoculated into an L medium, and it was then cultured at 30°C for 2 days. Thereafter, genomic DNA was extracted from the cell mass obtained in the same manner as that of Example 1. A fragment comprising a CYPSS204A1 gene and a ferredoxin gene located downstream thereof was amplified from the above-described genomic DNA by using a primer CYP-5F (SEQ ID NO: 9) and a primer CYP-6R (SEQ ID NO: 10) and also using La Taq polymerase (manufactured by Takara Bio INC.). The temperature conditions applied during this operation were 94 C/3 min, 30 cycles of (98 °C/20 sec,. 63 °C/30 sec, and 68 °C/2 min), and 72 °C/5 min. The nucleotide sequence of the amplified 1.8-kb DNA product is shown in SEQ ID NO: 4. In this nucleotide sequence, nucleotides 1 to 1422 correspond to the CYP204A1, and nucleotides 1433 to 1765 correspond to the ferredoxin gene.

A 1.8-kb DNA fragment amplified as a result of this reaction was purified using QlAquick PCR purification Kit (manufactured by QIAGEN), and the resultant was then digested with Nde I and Spe 1. Using T4 DNA ligase, the digest was ligated to an *Escherichia coli* expression vector pT7-camAB, and *Escherichia coli* DH5alpha was then transformed with the expression vector, so as to construct a plasmid pCYF204A1-camAB. *Escherichia coli* BL21(DE3) was transformed with this plasmid, and the obtained cell strain was named as BP172. The amino acid sequence encoded by the CYP204A1 gene is shown n SEQ ID NO: 23.

### Example 4: Expression of CYP204A1 gene in Bacillus megaterium and conversion of 4-cholesten-3-one to progesterone with use of this cell mass

The pCYP204A1-camAB produced in Example 3 was used as a template, and a fragment comprising the CYP204A1 gene and the ferredoxin gene located downstream thereof was amplified using primers CYP-7F (SEQ ID NO: 11) and CYP-8R (SEQ ID NO: 12) and KOD plus polymerase (manufactured by TOYOBO Co., Ltd.). The temperature conditions applied during this operation were 94°C/2 min, 20 cycles of (94 °C/20 sec, 55 °C/30 sec, and 68 °C/3 min), and 72 °C/5 min.

A 1.8-kb DNA fragment amplified as a result of this reaction, which comprised the CYP204A1 gene and the ferredoxin gene located downstream thereof was purified using QIAquick

PCR purification Kit (manufactured by QIAGEN), and the resultant was then digested with Nhe I and Barn HI. Thereafter, using T4 DNA ligase, the digest was ligated to *Bacillus megaterium* expression vector pHW1520 (manufactured by MoBiTec), and *Escherichia coli* DH5alpha was then transformed with the expression vector, so as to construct a plasmid pXYL204CB. *Bacillus megaterium* competent cells (manufactured by MoBiTec) were transformed with this plasmid, so as to obtain a cell strain pXYL204CB.

This cell strain was inoculated into 2 ml of TB medium containing tetracycline (final concentration: 1.0 mg/ml), and it was then subjected to shaking culture at 37 °C at 220 rpm for 16 hours. Hereafter, 250 µl of the obtained pre-culture was added to 25 ml of the same type of TB medium, and it was then subjected to shaking culture at 37 °C for 2.5 hours. Subsequently, 250 µl of 50 % xylose was added to the culture solution, and the mixture was further subjected to a shaking culture at 30 °C at 125 rpm for 6 hours. The cell mass recovered from the obtained culture by centrifugation was suspended in 5 ml of buffer for conversion (50 mM KPB, 2 % glycerol [pH 7.4]) to prepare a cell suspension.

Thereafter, 2 µl of solution of 4-cholesten-3-one in methanol (100 mg/ml) was added to 2 ml of the cell suspension, and the obtained mixture was then incubated at 30 °C for 24 hours while shaking (220 rpm). Then, 2 ml of ethyl acetate was added to the reaction solution, and the obtained mixture was then shaken with the use of a vortex, followed by centrifugation at 4,000 rpm for 10 minutes. The obtained ethyl acetate phase (1.5 ml) was solidified by concentration, it was then dissolved in 200 µl of methanol, and the obtained solution was then subjected to HPLC analysis. As a result of the analysis, it was found that 4-cholesten-3-one was converted to progesterone at a conversion rate of 24.5 %. HPLC conditions were as follows: Column: Inertsil ODS-3 4.6 x 50 mm, flow rate: 1.2 ml/min, temp.: 40°C, detect: PDA (240 nm), gradient: 0/4/5/5.5 (main), 40/100/100/40 (%B).

### Example 5: Properties of enzyme protein

### (1) Construction of expression vector

In order to amplify DNA fragments each comprising a CYP204A1, CYPSS or CYP11A gene, the following primers were produced: CYP204A1-1F (SEQ ID NO: 13). CYP204A1-2R (SEQ ID NO: 14), CYPSS-1F (SEQ ID NO: 15), CYPSS-2R (SEQ ID NO: 16), CYP11A-1F

(SEQ ID NO: 17), CYP11A-2R (SEQ ID NO: 18). Subsequently, pCYP204A1-camAB, pCYPSS-camAB, and pCYP11A-ARX (see Reference Example 1) into which CYP11A had been inserted as bovine-derived P450scc, were used as templates, and PCR reaction was carried out using the aforementioned primers and KOD plus (TOYOBO). The temperature conditions applied during this reaction were 95 °C/3 min, 25 cycles of (98 °C/20 sec, 55 °C/30 sec, and 72 °C/2 min), and 72 °C/5 min.

As a result of this reaction, approximately 1.4-kbp DNA fragments (hereinafter referred to as DNA fragments A, B and C. respectively) were amplified from CYP204A1, CYPSS and CYP11A. These DNA fragments were purified using Wizard SV Gel and PCR Clean-Up System (PROMEGA) and were then digested with NdeI and XhoI. Then, using DNA Ligation Kit ver 2.1 (TaKaRa), each digest was ligated to pET22b, so as to obtain plasmids pET22-CYP204A1 pET22-CYFSS, and pET22-CYP11A.

### (2) Production of CYF204A1-, CYPSS204A- and CYP11A-induced cell masses

*Escherichia coli* BL21 (DE3) was transformed with each of the plasmids pET22-CYP204A1, pET22-CYPSS, and pET22-CYP11A prepared in (1) above. Each of the thus obtained transformants was inoculated into 1 ml of TB medium containing 50 µg/ml carbenicillin, and it was then cultured at 37 °C for 14 hours. Thereafter, 1 ml of the culture solution was inoculated into 100 ml of TB medium containing 50 µg/ml carbenicillin and Overnight Express Autoinduction system 1 (Merck), followed by a culture at 25 °C for 24 hours and induction of proteins of interest. Each cell mass was recovered by centrifugation, and it was then suspended in 20 ml of CV buffer (50 mM KPB (potassium phosphate buffer), 10 % glycerol, 1 mM EDTA, 2 mM dithiothreitol, and 1 mM D-glucose). Then, the cell suspension was conserved at -80 °C.

### (3) Preparation of cell-free extract

To 20 ml of the cell suspension prepared in (2) above, 680 µl of X10 Bug Buster (Novagen), 20 µl of Benzonase (Novagen), and 2 ml of 40 mg/ml lysozyme were added, and the obtained mixture was then shaken at 30 °C for 30 minutes to disintegrate the cells. Thereafler, the reaction solution was centrifuged to obtain the cell-free extract. The obtained, approximately 20 ml of cell-free extract was dialyzed against 1 L of Buffer R (20 mM KPB pH 7.4, 20 % glycerol) two times, and the deposited precipitate was then removed by centrifugation. The cell-free extract obtained as a supernatant herein was used in the subsequent experiments.

### (4) Confirmation of expression of various types of P450scc by spectral analysis of carbon monoxide binding

The obtained cell-free extract was divided into two portions, and one portion was bubbled with carbon monoxide. Dithionite was added to the two samples, to which carbon monoxide bubbling had been or had not been performed, and difference spectrum was then measured. A peak having maximum absorption around 450 nm was obtained in the case of CYP204A1 and CYPSS204A, and thus, the expression of P450scc was confirmed. On the other hand, in the case of CYP11A, such a peak could not be obtained, and thus, it was suggested that no active P450 would be present in the sample. Based on these results, the subsequent experiments were carried out using CYP204A1 and CYPSS.

### (5) Analysis of the optimum pH

Using the cell-free extract prepared in (3) above, conversion of 4-cholesten-3-one was carried out at various pH values. The reaction solution was prepared, such that 1 ml of Buffer R comprised 215 pmol/ml CYP204A1 or 367 pmol/ml CYPSS204A, 64 µg/ml spinach-derived ferredoxin, a 0.1 U/ml spinach-derived ferredoxin reductase protein, 3 U/ml glucose dehydrogenase, 60 mM glucose, 50 µg/ml 4-cholesten-3-one, 2 mM NADH, and 2 mM NADPH. Then, 562 µl of 200 mM Buffer having various pH values was added to 1 ml of the reaction solution, so as to adjust the pH of the reaction solution. The reaction was initiated by the addition of NADH and NADPH, and it was carried out at 200 rpm at 30°C for 14 hours. Thereafter, 1.5 ml of ethyl acetate was added to the reaction solution to terminate the reaction, and extraction was carried out. Then, extraction was carried out again with 0.75 ml of ethyl acetate. The obtained ethyl acetate phase was solidified using evaporator, and the residue was then dissolved in 200 µl of methanol. Thereafter, progesterone was detected by HPLC analysis. The enzyme protein activity for conversion of 4-cholesten-3-one to progesterone under various pH conditions is shown in Table 1. The enzyme protein activity was indicated by the amount (g) of progesterone generated per hour per mol of P450. The results demonstrated that CYP204A1 and CYPSS had the highest activity at pH 7.5 of Tris-HCl.

### (6) Analysis of the optimum temperature

Using the cell-free extract prepared in (3) above, conversion of 4-cholesten-3-one was carried out at various pH values. The reaction solution was prepared, such that 1 ml of the buffer comprised 215 pmol/ml CYP204A1 or 391 pmol/ml CYPSS, 64 µg/ml spinach-derived ferredoxin, 0.1 U/ml spinach-derived ferredoxin reductase protein, 3 U/ml glucose dehydrogenase, 60 mM glucose, 50 µg/ml 4-cholesten-3-one, 2 mM NADH. and 2 mM NADPH. Tris-HCl was added to the reaction solution so as to adjust it to pH 7.5. The reaction was initiated by addition of NADH and NADPH, and it was carried out at 200 rpm for 4 hours, under temperature conditions of 10°C, 15°C, 20'C. 25°C, or 30°C. Thereafter, 1.5 ml of ethyl acetate was added to the reaction solution to terminate the reaction, and extraction was carried out. Then, extraction was carried out again with 0.75 ml of ethyl acetate. The obtained ethyl acetate phase was solidified using evaporator, and the residue was then dissolved in 200 µl of methanol. Thereafter, progesterone was detected by HPLC analysis. The enzyme protein activity for conversion of 4-cholesten-3-one to progesterone under various temperature conditions is shown in Table 1. The results demonstrated that CYP204A1 had the highest activity from 15 °C to 20°C and CYPSS had the highest activity at 15 °C.

**Table 1:**

| · ***Optimum pH*** Buffer | pH | Specific activity CYP204A1 | (g/hr/mol) CYPSS |
|---|---|---|---|
| KPB | 6.2 | 0 | 0 |
| | 6.7 | 0 | 19 |
| | 7.1 | 0 | 21 |
| | 7.4 | 0 | 0 |
| | 7.9 | 0 | 0 |
| Tris-HCl | 7.5 | 207 | 301 |
| | 8.2 | 114 | 269 |
| | 8.7 | 0 | 40 |
| TES | 6.6 | 0 | 0 |
| | 6.8 | 0 | 16 |
| | 7.3 | 39 | 76 |
| | 7.5 | 97 | 165 |
| | 8.0 | 82 | 168 |

| · ***Optimum temperature*** Temp. (°C) | | Specific activity CYP204A1 | (g/hr/mol) CYPSS |
|---|---|---|---|
| 10 | | 507 | 918 |
| 16 | | 698 | 1279 |
| 20 | | 719 | 1133 |
| 25 | | 550 | 936 |
| 30 | | 285 | 465 |

### (7) pH stability test

0.5 ml of the cell-free extract prepared in (3) above was mixed with 0.5 ml of 50 mM buffer having various pH values, pH was then measured, and the mixture was then conserved at 6°C for 140 hours. The pH values of the enzyme protein solution were: AcB (acetate buffer), pH 6.0; KPB, pH 6.7; KPB, pH 7.2; KPB, pH 7.6; TES, pH 7.5; Tris-HCl, pH 8.1; and Tris-HCl, pH 8.9. Using the prepared cell-free extracts having various pH values, conversion of 4-chalesten-3-one was carried out. The reaction was carried out, immediately after the enzyme protein solution was mixed with buffers having various pH values and after the mixed solution was conserved for 140 hours, so as to examine the stability. The reaction solution was prepared, such that 1 ml of the buffer comprised an enzyme protein solution of CYP204A1 or CYPSS having various pH values, 64 µg/ml spinach-derived ferredoxin, 0.1 U/ml spinach-derived ferredoxin reductase protein, 3 U/ml glucose dehydrogenase, 60 mM glucose, 50 µg/ml 4-cholesten-3-one, 2 mM NADH, and 2 mM NADPH. To the reaction solution, 562 µl of 200 mM Tris-HCl was added, so as to adjust the reaction solution to pH 7.5. It was conformed that the reaction solution maintained pH 7.5, even though enzyme protein solutions having various pH values were added thereto. The reaction was initiated by addition of NADH and NADPH, and it was carried out at 200 rpm at 15 °C for 4 hours. Thereafter, 1.5 ml of ethyl acetate was added to the reaction solution to terminate the reaction, and extraction was carried out. Then, extraction was carried out again with 0.75 ml of ethyl acetate. The obtained ethyl acetate phase was solidified using an evaporator, and the residue was then dissolved in 200 µl of methanol. Thereafter, progesterone was detected by HPLC analysis. The enzyme activity for conversion of 4-cholesten-3-one to progesterone at 0 hour and 140 hours is shown in Table 2. The results demonstrated that both CYP204A1 and CYPSS were most stable at KPB, pH 7.6.

**Table 2**

| **CYP204A1** | **activity (µg/ml)** | | |
|---|---|---|---|
| Sample | 0 hr | 140 hr | Relative activity(%) |
| AcB Ph6.0 | 2.1 | 0.5 | 24 |
| KPB pH 6.7 | 1.9 | 1 | 53 |
| KPB pH 7.2 | 2 | 1.3 | 65 |
| **KPB pH 7.6** | **2** | **1.4** | **70** |
| TES pH7.5 | 2.2 | 1.5 | 68 |
| Tris pH 7.4 | 2.1 | 1.3 | 62 |
| Tris pH 8.1 | 2 | 1 | 50 |
| Tris pH 8.9 | 2 | 0.7 | 35 |
| | | | |
| | | | |

| **CYPSS** | **Activity (µg/ml)** | | |
|---|---|---|---|
| Sample | 0 hr | 140 hr | Relative activity (%) |
| AcB pH 6.0 | 7.3 | 2.7 | 37 |
| KRB pH 6.7 | 7.1 | 4.3 | 61 |
| KPB pH 7.2 | 7.3 | 5.3 | 73 |
| **KPB pH 7.6** | **6.5** | **5.5** | **8.5** |
| TES pH 7.5 | 7.3 | 5.2 | 71 |
| Tris pH 7.4 | 8 | 5 | 63 |
| Tris pH 8.1 | 6.9 | 4.5 | 65 |
| Tris pH 8.9 | 7.3 | 3.7 | 51 |

### (8) Thermostability test

The cell-free extract prepared in (3) above was conserved at various temperature for 140 hours. The conservation temperature was set at 6 °C, 10°C, 20 °C, 30 °C, 37 °C, or 45 °C. Using the enzyme protein solution before conservation and the sample obtained after conservation for 140 hours, conversion of 4-cholesten-3-one was carried out, and stability was examined. The reaction solution was prepared, such that 1 ml of the buffer comprised an enzyme protein solution of CYP204A1 or CYPSS that had been conserved at each temperature, 64 µg/ml spinach-derived ferredoxin, 0.1 U/ml spinach-derived ferredoxin reductase protein, 3 U/ml glucose dehydrogenase, 60 mM glucose, 50 µg/ml 4-cholesten-3-one, 2 mM NADH, and 2 mM NADPH. The reaction was initiated by addition af NADH and NADPH, and it was carried out at 200 rpm at 15°C for 4 hours. Thereafter. 1.5 ml of ethyl acetate was added to the reaction solution to terminate the reaction, and extraction was carried out. Then, extraction was carried out again with 0.75 ml of ethyl acetate. The obtained ethyl acetate phase was solidified using an evaporator, and the residue was then dissolved in 200 µl of methanol. Thereafter, progesterone was detected by HPLC analysis. The enzyme protein activity for conversion of 4-cholesten-3-one to progesterone at 0 hour and 140 hours is shown in Table 3. The results demonstrated that, in both cases of CYP204A1 and CYPSS, stability increased as the conservation temperature decreased. In particular, when the cell-free extract was conversed at 20 °C for 140 hours, CYP204A1 maintained 47 % of its activity and CYPSS maintained 39 % of its activity.

**Table 3**

| **CYP204A1 Activity** | **(µg/ml)** | | |
|---|---|---|---|
| Temperature | 0 hr | 140 hr | Relative activity (%) |
| **6°C** | **5.3** | **4.5** | **85** |
| 10°C | 5.3 | 3.9 | 74 |
| 20°C | 5.3 | 2.5 | 47 |
| 30°C | 5.3 | 0.5 | 9 |
| 37°0 | 5.3 | 0 | 0 |
| 45°C | 5.3 | 0 | 0 |

| **CYPSS** | **Activity (**µ**g/ml)** | | |
|---|---|---|---|
| Temperature | 0 hr | 140 hr | Relative activity (%) |
| **6°C** | **14.4** | **11.6** | **81** |
| 10°C | 14.4 | 10.5 | 73 |
| 20°C | 14.4 | 5.6 | 39 |
| 30°C | 14.4 | 1 | 7 |
| 37°C | 14.4 | 0 | 0 |
| 45°C | 14.4 | 0 | 0 |

### (9) Kinetics analysis of CYP204A1 and CYPSS

Using the cell-free extract prepared in (3) above, conversion of 4-cholesten-3-one was carried out at substrate concentration of 0.1 to 500 µM, and the relationship between the substrate concentration and the enzyme protein activity was examined. The reaction solution was prepared, such that 1 ml of the buffer comprised 220 pmol CYP204A1 or 250 pmol CYPSS, 96 µg/ml spinach-derived ferredoxin, 0.1 U/ml spinach-derived ferredoxin reductase protein, 3 U/ml glucose dehydrogenase, 60 mM glucose, 2 mM NADH, and 2 mM NADPH. Tris-HCl was added to the reaction solution so as to adjust it to pH 7.5. 20 µl of 4-cholesten-3-one, which had been dissolved in DMSO to a final concentration of 0.1, 0.5, 1, 2, 5, 10, 20, 50, 100 or 500 µM, was added. The reaction was initiated by addition of NADH and NADPH, and it was carried out at 200 rpm at 15 °C for 60 minutes. Thereafter, 1.5 ml of ethyl acetate was added to the reaction solution to terminate the reaction, and extraction was carried out. Then, extraction was carried out again with 0.75 ml of ethyl acetate. The obtained ethyl acetate phase was solidified using evaporator, and the residue was then dissolved in 200 µl of methanol. Thereafter, progesterone was detected by HPLC analysis. The relationship between the substrate concentration and the enzyme protein activity, and kinetics parameters calculated by production of Lineweaver-Burk plot, is shown in Table 5. The results demonstrated that CYP204A1 and CYPSS had similar Km values (1.0 and 1.1 µM), and that their Vmax were 47.4 and 78.7 mmol/min/mol, respectively, thereby showing that the Vmax value of CYPSS was approximately 1.7 times higher than that of CYP204A1. In addition, the comparison of these results demonstrated that CYPSS hardly undergoes substrate inhibition.

**Table 4**

| ***• Kinetics*** 4Cholesten-3-one | Specific activity (mmol/min/mol) | | | |
|---|---|---|---|---|
| concn. (µM) | CYP204A1 | | CYPSS | |
| 0.1 | 2.2 | | 1.9 | |
| 0.5 | 15.3 | | 20.0 | |
| 1 | 25.2 | | 36.5 | |
| 2 | 32.2 | | 49.5 | |
| 5 | 36.1 | | 70.4 | |
| 10 | 43.1 | 100 | 67.2 | 100 |
| 20 | 42.7 | | 72.4 | |
| 50 | 33.5 | | 71.2 | |
| 100 | 28.7 | 66.6 | 60.5 | 90.0 |
| 500 | 20.1 | | 33.6 | |

**Table 5**

| **Kinetics parameters** | | | |
|---|---|---|---|
| | | Vmax | Km |
| | CYP204A1 | 47.4 (1.00) | 1.0 |
| | CYPSS | 78.7 (1.66) | 1.1 |
| | | mmol/min/mol | µM |
| | | (): Relative activity | |

The summary of the above-described results is shown below. The optimum pH: pH 7.5 to 8.0 (in both cases of CYP204A1 and CYPSS); the optimum temperature for action: 15 °C to 20 °C (in both cases of CYP204A1 and CYPSS); pH stability: stable around the neutral range, and most stable at KPB pH 7.6 (in both cases of CYP204A1 and CYPSS); thermostability: 30 % or more of enzyme protein activity that was retained after conversion at 20 °C for 140 hours (in both cases of CYP204A1 and CYPSS); kinetic analysis: Vmax of CYPSS that was approximately 1.7 times higher than that of CYP204A1, and Km value of CYPSS that was almost equivalent to that of CYP204A1 (1 µM). Moreover, regarding substrate inhibition, it was found that 4-cholesten-3-one causes substrate inhibition, and that substrate inhibition of CYPSS was lower than that of CYP204A1. Furthermore, in comparison with the enzyme protein activity of CYPSS or CYP204A1 when the concentration of 4-cholesten-3-one was 10 µM, the enzyme protein activity when it was 100 µg/ml was 90 % or 67 %. That is to say, when the substrate concentration was increased from 10 µM to 100 µM, the decrease rate of the enzyme protein activity was 10 % or 33 %.

### Example 6: Conversion reaction of 4-cholesten-3-one by strain BP215 (preparation of progesterone)

The *Escherichia coli* expression strain BP215 was inoculated into 2 ml of an M9 mix medium (Na2HPO4: 0.68 %, KH2PO4: 0.3 %, NaCl: 0.05 %, NH4Cl: 0.01 %, Casamino acid: 1 %, D-Glucose: 0.4 %, CaCl2: 0.1 mM, MgCl2: 1 mM, Thiamine: 0.002 %, FeSO4: 0.1mM, Carbenicillin: 0.005 %), and it was then subjected to shaking culture at 30 °C for 17 hours, so as to obtain pre-culture solution. 0.25 ml of this pre-culture solution was inoculated into 25 ml of the main culture medium (Na2HPO4: 0.68 %, KH2PO4: 0.3 %, NaCl: 0.05 %, NH4Cl: 0.01 %, Casamino acid: 1 %, CaCl2: 0.1 mM, MgCl2: 1mM, Thiamine: 0.002 %, FeSO4: 0.1 mM, Carbenicillin: 0.005 %, Overnight Express^{™} solution: 7 %, 5-Aminolevlic acid: 0.008 %) using Overnight Express^{™} Autoinduction System (Novagen Cat. No. 71300-4), and it was then subjected to shaking culture at 25 °C for 24 hours. 5 ml of the obtained main culture solution was centrifuged (3500 rpm, 10 minutes) to obtain cell mass, and 1 ml of CV2 buffer (50 mM (pH) potassium phosphate buffer containing glycerol: 2 %, carbenicillin: 0.005 %, and isopropyl β-D-1-thiogalactopyranoside: 0.1 mM) was added to the obtained cell mass. Thereafter, 0.01 ml of the methanol solution of 1 % 4-cholesten-3-one and 0.02 ml of an aqueous solution of 25 % methyl-β-cyclodextrin were added to the mixed solution, and the obtained mixture was then shaken at 28 °C for 5 hours for the reaction. After completion of the reaction, 4 ml of methanol was added to the reaction solution, and they were then fully mixed. The obtained mixture was centrifuged, and the upper layer was then analyzed by HPLC. The peak of progesterone was detected at retention time of 2.6 minutes, and thus, the progesterone preparation, the retention time, and the MS spectrum (m/z = 315 M + 1) were matched. The accumulated amount was found to be 78.0 mg/l (conversion rate: 78 %). On the other hand, 4-cholesten-3-one (retention time: 8.1 minutes) was not detected. Analysis conditions: Using Chromolith speed rod RP18e (50 × 4.6 mm) column, 50 % →100 % CH3CN gradient (0 to 3 min) and 100 % CH3CN (3 to 8 min) were used as a mobile phase, and detection was carried out at a flow rate of 2 ml/min using CAD^{™} (Charged Aerosol Detection) and
MS.

### Example 7: Conversion reaction of cholesterol by strain BP215

Using cholesterol as substrate, the same reaction as that of Example 6 was carried out, and treatment and analysis were then carried out. As a result, the peak of pregnenolone was detected at retention time of 2.5 minutes, and thus, the pregnenolone preparation, the retention time, and the MS spectrum (m/z = 317 M + 1) were matched. The accumulated amount was found to be 68.2 mg/l (conversion rate: 68.2 %). On the other hand, 3.7 % of cholesterol (retention time: 8.5 minutes) remained.

### Example 8: Conversion reaction of 7-dehydracholesterol and ergosterol by strain BP215

Using 7-dehydrocholesterol as substrate, the same reaction as that of Example 6 was carried out, and treatment and analysis were then carried out. As a result, the peak of 7-dehydropregnenolone (m/z = 315 M + 1) was detected at retention time of 2.1 minutes. The accumulated amount was found to be 34.3 mg/l (conversion rate: 34.3 %). On the other hand, 54 % of 7-dehydrocholesterol (retention time: 7.5 minutes) remained.

Using ergosterol as substrate, the same reaction as that of Example 6 was carried out, and treatment and analysis were then carried out. As a result, the peak of 7-dehydropregnenolone was detected at a retention time of 2.1 minutes. The accumulated amount was found to be 2.8 mg/l (conversion rate: 2.8 %).

### Example 9: Conversion reaction of various types of substrates by strain BP215

As substrate, β-sitosterol, stigmasterol, campesterol or desmosterol was used. Each substrate was subjected to the same reaction as that of Example 6, separately, and the treatment and the analysis were then carried out. As a result, the peak of pregnenolone was detected at the retention time of 2.5 minutes, and thus, the pregnenolone preparation, the retention time, and the MS spectrum (m/z = 317M + 1) were matched. The accumulated mounts were found to be 40.9 mg/l (conversion rate: 40.9 %), 5.8 mg/l (conversion rate: 5.8 %), 27.9 mg/l (conversion rate: 27.9 %), and 71.7 mg/l (conversion rate: 71.7 %), respectively. On the other hand, the added substrate (the retention time was 10.1, 9.3, 9.2, and 7.2 minutes, respectively) remained at 55.9 %, 70.8 %, 25.5 %, and 21.3 %, respectively. It is to be noted that since the product generated from β-sitosterol was in an extremely small amount, the reaction solution was extracted with ethyl acetate, was then concentrated by a factor of 5, and was then subjected to the analysis.

### Example 10: Chemical synthesis of (20R,22R)-20,22-dihydroxy-4-cholest-4-en-3-one and (20R,22S)-20,23-dihydroxycholest-4-en-3-one

The synthesis of two stereoisomers of 20,22-dihydroxycholest-4-en-3-one was carried out by going through 20,22-dihydroxycholesterol according to the synthesis method described in the known publication, and then applying an ordinary oxidation reaction.

### (A) Synthesis of 20,22-dihydroxycholesterol (10-A)

The chemical synthesis of the two isomers [a (20R,22R) form and a (20R,22S) form] of the compound (10-A) is described in Steroids (2004), 69, 483/B. Watanabe et al. Thus, the compound (10-A) was synthesized in accordance with the experimental methods described in the above-mentioned publication.

The essence of the synthesis is that pregnenolone was used as a starting material, the group at position 3 thereof was protected by t-butyldimethylsilyl chloride, and 4-methyl-1-pentine was added thereto to obtain (20R)-3β-(t-butyldimethylsilyloxy)cholest-5-en-22-yn-20-ol. This compound was reduced to cis-olefin, using Lindlar catalyst, so as to obtain (20R,22Z)-3β-(t-butyldimetliylsilyloxy)cholest-5,22-dien-20-ol. This compound was converted to 22,23-epoxide (an isomeric mixture) by VO(acac)2/TBHP, and it was kept as an isomeric mixture without being separated. It was reduced by LiAlH4, and was converted to 20,22-dihydroxy form. Thereafter, the group at position 3 thereof was deprotected by TBAF, so as to obtain (20R,22RS)-20,22-dihydroxycholesterol (10-A).

### (B) Synthesis of 20,22-dihydroxycholest-4-en-3-one (10-B)

With reference to the method described in the above-mentioned publication, (20R,22RS)-20,22-O-isopropylidenedioxycholesterol was first obtained from the compound (10-A) [wherein 22-dimethoxypropane was allowed to act thereon in the presence of PPTS]. The obtained compound was converted to the 4-en-3-one form by Oppenauer oxidation according to an ordinary method. The experimental procedures will be specifically described below.

(20R,22RS)-20,22-O-isoprppylidenedioxycholesterol (3.0 g) was dissolved in 100 mL of toluene in Dean-Stark apparatus that had been sufficiently subjected to nitrogen substitution, and 1-methyl-4-piperidone (8.1 mL, 10 eq) was then added to the solution Thereafter, the reaction temperature was set at 130°C to 140°C, Al(OiPr)3 (2.7 g, 2 eq) was then added to the reaction solution, and the obtained mixture was then stirred for 4 hours. After completion of the reaction, the reaction solution was diluted with diethyl ether, was then subjected to ordinary treatments, and was then purified by silica gel chromatography [hexane/ethyl acetate = 8/1 to 5/1], so as to obtain (20R,22RS)-20,22-O-isopropylidenedioxycholest-4-en-3-one (2.9 g, y 97 %).

Finally, (20R,22RS)-20,22-O-isopropylidenedioxycholest-4-en-3-one was converted to the compound (10-B) by deprotecting acetonide, and further, two stereoisomers could be obtained, separately. Specific experimental procedures will be described below.

(20R,22RS)-20,22-O-isopropylidenedioxycholest-4-en-3-one (2.9g) was stirred at room temperature in THF (20 mL) + methanol (20 mL). In the mean time, 2 N hydrochloric acid (1.0 mL) and perchloric acid (0.8 ml) were added thereto, and the reaction was then carried out for 60 hours. Then, the reaction solution was diluted with chloroform and was then subjected to ordinary treatments. Thereafter, the reaction solution was purified by silica gel chromatography [hexane/ethyl acetate = 7/1 to 2/1], so that a deprotected product (1.48 g, y 47%) was separated from an unreacted raw material (1.24 g), and thus they were obtained, separately. A white solid (1.1 g) was obtained from the deprotected product by recrystallization from hexane/ethyl acetate. 1H-NMR(400MHz,CDCl3) δ: 0.90(d,J = 6.6Hz,26-H₃), 0.90(d,J = 6.8Hz,27-H₃), 0.91(s,18-H₃), 1.19(s,19-H₃), 1.26(s,21-H₃), 3.24(dd,J = 9.4Hz,5.4Hz,22-H), 5.73(s,4-H) → (20R,22S)-20,22-dihydroxycholest-4-en-3-one (10-B) [non-natural isomer, which is a novel compound obtained by the present invention]

The recovered unreacted raw material (590 mg) was stirred at room temperature in THF (15 mL) + methanol (15 mL). In the meantime, perchloric acid (1.4 mL) was added thereto, and the reaction was then carried out for 10 hours. Then, the reaction solution was diluted with chloroform and was then subjected to ordinary treatments. Thereafter, the reaction solution was purified by silica gel chromatography [hexane/ethyl acetate = 7/1 to 2/1], so as to obtain a deprotected product (217 mg, y 40%).
1H-NMR(500MHz,CDCl3) δ: 0.89(d,J = 6.3Hz,26-H₃), 0.91(d,J = 6.4Hz,27-H₃), 0.93(s,18-H₃), 1.19(s,19-H₃), 1.21(s,21-H₃), 3.38(d,J = 8.4Hz,22-H), 5.73(s,4-H) → (20R,22R)-20,22-dihydr oxycholest-4-en-3-one (10-B) [natural isomer; (citation) Helv. Chim. Acta, (2006), 89, 813/W. Zhang et al.]

### Example 11: Conversion reaction of various types of substrates by strain BP215

As substrates, (20S)-20-hydroxycholest-4-en-3-one, (22R)-22-hydroxycholest-4-en-3-one, (20R,22R)-20,22-dihydroxycholest-4-en-3-one, and (20R,22S)-20,22-dihydroxycholest-4-en-3-one were used. Each substrate was subjected to the same reaction as that of Example 6, separately, and the generated product was then analyzed by HPLC. As a result, generation of progesterone was confirmed. The conversion rates were found to be 41.0 %, 42.3 %, 38.1 % and 69.8 %, respectively. On the other hand, the added substrates (their retention times were 4.9, 4.4, 3.8, and 3.5 minutes, respectively) remained at 4.8 %, 2.7 %, 0.6 %, and 0.6 %, respectively.

Moreover, as substrates, (20S)-20-hydroxy-cholesterol, (22S)-22-hydroxy-cholesterol. (22R)-22-hydroxy-cholesterol, and (20R,22R)-20,22-dihydroxy-cholesterol were used. Each substrate was subjected to the same reaction as that of Example 6, separately, and treatment and analysis were then carried out. As a result, generation of pregnenolone as a side-chain cleavage body corresponding to each substrate was confirmed. The accumulated amounts were found to be 89.2 mg/L (conversion rate: 89.2 %), 37.5 mg/L (conversion rate: 37.5 %), 80.0 mg/L (conversion rate: 80 %), and 53.0 mg/L (conversion rate: 53.0 %), respectively. On the other hand, the added substrates remained at 0 %, 23.5 %, 14.8 %, and 0 %, respectively.

### Example 12: Conversion reaction of various types of substrates by strain BP172

As substrates, 4-cholesten-3-one, cholesterol, 7-dehydrocholesterol, ergosterol, β-sitosterol, stigmasterol, campesterol, desmosterol, (20S)-20-hydroxycholest-4-en-3-one, (22R)-22-hydroxycholest-4-en-3-one, (20R,22R)-20,22-dihydroxycholest-4-en-3-one, and (20R,22S)-20,22-dihydroxycholest-4-en-3-one were used. Each substrate was subjected to the same reaction as that of Example 6, separately, and the generated product was then analyzed by HPLC. As a result, generation of progesterone, pregnenolone or 7-dehydropregnenolone as a side-chain cleavage body corresponding to each substrate was confirmed. The conversion rates were found to be 84.0 %, 71.2 %, 28.5 %, 22 %, 47.2 %, 6.5 %, 31.3 %, 66.1 %, 63.9 %, 80.9 %, 61.3 % and 41.7 %, respectively.

Moreover, as substrates, (20S)-20-hydroxy-cholesterol, (22S)-22-hydroxy-cholesterol, (22R)-22-hydroxy-cholesterol, and (20R,23R)-20,22-dihydroxy-cholesterol were used. Each substrate was subjected to the same reaction as that of Example 6, separately, and treatment and analysis were then carried out. As a result, generation of pregnenolone as a side-chain cleavage body corresponding to each substrate was confirmed. The accumulated amounts were found to be 68.2 mg/L (conversion rate: 68.2 %), 12.9 mg/L (conversion rate: 12.9 %), 69.5 mg/L (conversion rate: 69.5 %), and 51.6 mg/L (conversion rate: 51.6 %), respectively. On the other hand, the added substrates remained at 25.2 %, 60.1 %, 42.8 % and 0 %, respectively.

### Reference Example 1: Production of CYP11A-expressing Escherichia coli

A commercially available human testis cDNA (Origene Technologies, Inc.) was used as a template, and a CYP11A1 gene was amplified by PCR using a primer CYP11A-1F (SEQ ID NO: 19) and a primer CYP11A-2R (SEQ ID NO: 20), and also using KOD plus polymerase (TOYOBO Co., Ltd.). The temperature conditions applied during the PCR were 94 °C/3 min, 30 cycles of (94 °C/30 sec, 55 °C/60 sec, and 72 °C/90sec), and 72 °C/5 min.

A 1.45-kb CYP11A1 gene fragment amplified as a result of the above-described reaction was purified using QIAquick PCR purification Kit (manufactured by QIAGEN), and it was then digested with Nde I and Spe I. Thereafter, the digest was ligated to *Escherichia coli* expression vector pT7NS-camAB. using T4 DNA ligase, and *Escherichia coli* DH5α was then transformed with the obtained expression vector, so as to construct a plasmid pCYP11A-camAB. Subsequently, using T4 DNA ligase, the previously prepared bovine-derived adrenodoxin reductase protein-adrenodoxin gene (adr-adx) fragment was inserted into and ligated to each of the Spe I and Bam HI sites of the above-described plasmid, and *Escherichia coli* DH5α was then transformed with the obtained plasmid, so as to construct plasmid pCYP11A-ARX. Thereafter, *Escherichia coli* BL21(DE3) was transformer with the thus constructed plasmid, and the obtained cell strain was named as BL21CYP11. The used adx-adr fragment was amplified by PCR with a plasmid pKARX (Sawada et. al. Eur. J. Biochem. 265, 950-956, 1999) as a template, using primer bAdxR-1F (SEQ ID NO: 21) and primer bAdx-2R (SEQ ID NO: 22), and also using KOD plus polymerase (manufactured by TOYOBO Co., Ltd.). The temperature condition applied during the PCR were 94 °C/3 min, 25 cycles of (94 °C/30 sec, 60 °C/30 sec, and 72 °C/120sec), and 72 °C/5 min.

A 1.85-kb adr-adx gene fragment amplified as a result of the above-described reaction was purified using QIAquick PCR purification Kit (manufactured by QIAGEN), and it was then digested with Spe I and Fba I. Thereafter, the digest was used to construct the above-described pCYP11A-ARX.

### Comparative Example: Conversion reaction of various types of substrates by CYP11A-expressing Escherichia coli

As substrate, 4-cholesten-3-one (4Chol), cholesterol (Chol), 7-dehydrocholesterol (7-DHC), ergosterol (Ergo), β-sitosterol (Sito), stigmasterol (Stigma), campesterol (Cam), desmosterol (Des), (20S)-20-hydroxycholest-4-en-3-one (20-OH), (22R)-22-hydroxycholest--4-en-3-one (22-OH), (20R,22R)-20,22-dihydroxycholest-4-en-3-one (R-diol), (20R,22S)-20,22-dihydroxycholest-4-en-3-one (S-diol), or lanosterol (Lano) was used. Each substrate was subjected to the same reaction as that of Example 6, separately, and the reaction solution was then subjected to liquid separation using 1 ml of ethyl acetate. The reaction product and the substrate were extracted, and were then analyzed by HPLC. As a result, generation of progesterone, pregnenolone or 7-dehydropregnenolone as the side-chain cleavage body corresponding to each substrate was confirmed. The conversion rates were found to be 0 %, 2.6 %, 0.7 %, 0 %, 0.8 %, 0 %, 1.0 %, 1.2 %, 0 %, 0.4 %, 4.3 % and 0 %, respectively. On the other hand, the added substrates remained at 100 %, 99 %, 99.2 %, 100 %, 99.7 %, 100 %, 99.1 %, 99.6 %, 100 %, 99.7 %, 95 % and 100 %, respectively.

Moreover, as substrate, (20S)-20-hydroxy-cholesterol, (22S)-22-hydroxy-cholesterol, (22R)-22-hydroxy-cholesterol, or (20R, 22R)-20,22-dihydroxy-cholesterol was used. Each substrate was subjected to the same reaction as that of Example 6, separately, and the treatment and the analysis was then carried out. As a result, generation of pregnenolone, as the side-chain cleavage body corresponding to each substrate was confirmed. The accumulated amount was found to be 16.3 mg/L (conversion rate: 16.3 %), 6.1 mg/L (conversion rate: 6.1 %), 28.0 mg/L (conversion rate: 28.0 %), and 32.3 mg/L (conversion rate: 32.3 %), respectively. On the other hand, the added substrates remained at 98.8 %, 92.6 %, 78.6 % and 51.6 %, respectively.

A summary of the conversion reaction rate to individual substrate is shown in the following Table 6.

**Table 6: Conversion rate (%)**

| | 4Chol | S-diol | R-diol | 20-OH | 22OH | Chol | Stigma | Lano | Sito | Cam | Des | 7-DHC | Ergo |
|---|---|---|---|---|---|---|---|---|---|---|---|---|---|
| CVP11 | 0 | 0 | 4.3 | 0 | 0.4 | 2.6 | 0 | 0 | 0.8 | 1 | 1.2 | 0.7 | 0 |
| BP172 | 84 | 41.7 7 | 61.3 | 63.9 | 80.9 | 71. 2 | 6.5 | 0 | 47.2 | 31. 3 | 66. 1 | 28.5 | 2.2 |
| BP215 | 78 | 69.8 | 38. 1 | 41 | 42.3 | 68.2 | 5.8 | 0 | 40.9 | 27.9 | 71.7 | 34.3 | 2.8 |

### Example 13: SDS-PAGE

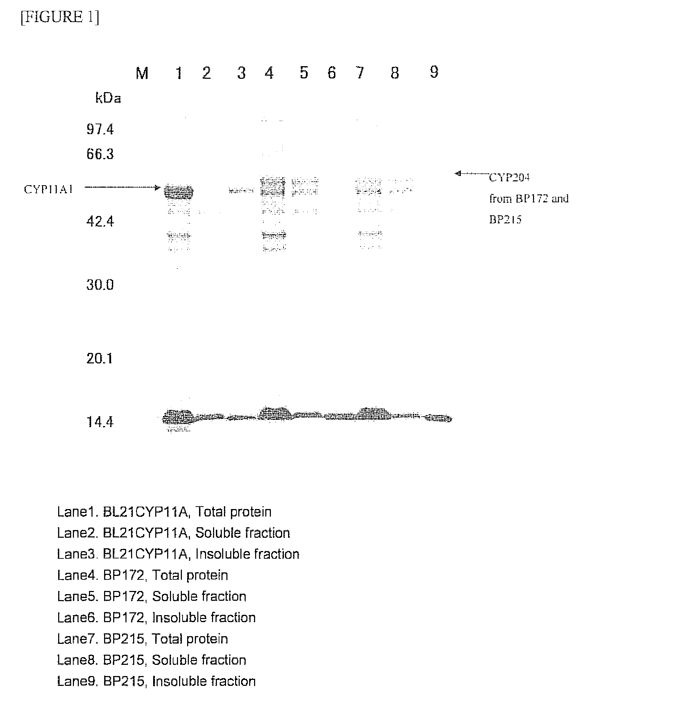

BL21CYP11A, BP172 and BP215 were each inoculated into 2 ml of TB medium comprising 50 µg/ml (the final concentration) ampicillin, and they were then subjected to shaking culture at 30 °C at 220 rpm for 16 hours. 250 µl of the obtained pre-culture was added to 25 ml of TB medium used for main culture (ampicillin 50 µg/ml <final concentration>, 5-aminolevulinic acid 40µg/ml <final concentration>, and inducer: Overnight Express Autoinduction System 1 <manufactured by Mercy>), and it was then subjected to the shaking culture at 25 °C for 24 hours. The cell mass recovered from the culture solution by centrifugation was suspended in 5 ml of buffer (50 mM KPB, 2% glycerol [pH 7.4]) so as to prepare the cell suspension. To 2 ml of this cell suspension, 66.7 µl of × 10 Bug Buster (manufactured by Novagen), 0.67 µl of Benzonase (manufactured by Novagen), and 40 mg/ml lysozyme were added, and the obtained mixture was then shaken at 30 °C for 20 minutes so that the cells were lysed. This cell lysis solution was defined as the total protein solution. Moreover, the cell lysis solution was centrifuged, and the obtained supernatant was defined as the soluble fraction protein solution. The insoluble fraction protein was prepared by centrifuging the cell lysis solution, completely removing the centrifuge supernatant, washing the residue with buffer 3 times, and then re-suspending the resultant in the buffer. These total protein solution, soluble fraction protein solution and insoluble fraction protein solution were each mixed with 20 µl of the SDS-PAGE sample buffer (manufactured by BIO-RAD), so as to prepare samples to be used for SDS-PAGE electrophoresis. The SDS-PAGE electrophoresis sample was heated at 95 °C for 5 minutes, and was then electrophoresed (300 V, 30 mA, 60 minutes) on polyacrylamide gel (manufactured by Daiichi Kagaku Yakuhin Kabushiki Kaisha). After completion of the electrophoresis, the polyacrylamide gel was transferred onto a tray, and an appropriate amount of staining-decolorating solution (manufactured by Takara Bio INC.) was then added thereto, so as to carry out staining and decoloration. The results are shown in Figure 1.

### Industrial Applicability

Using the novel enzyme protein of the present invention, it became possible to efficiently produce pregnenolone and the like and hydrocortisone, which are compounds industrially useful as medicaments and pharmaceutical intermediates.

### Brief Description of the Drawings

Figure 1 is a view showing the results of SDS-PAGE using BL21CYP11A, BP172 and BP215.
Figure 2 is a view showing derivatives of hydrocorticoid and a synthetic method thereof.
   1. Faming Zhuanli Shenqing Gongkai Shuomingshu, 1896090, 17 Jan 2007
   2. J. Am Chem. Soc., 82, 3696-701; 1960
   3. Journal of Steroid Biochemistry and Molecular Biology, 87(4-5), 319-325; 2004; and Annals of the New York Academy of Sciences, 434 (Enzyme Eng.), 106-9; 1984 Ger. Offen., 3322120, 02 Feb 1984
   4. Ger. Offen., 3109459, 23 Sep 1982; Journal of the American Chemical Society, 81, 4956-62; 1959; and Journal of the American Chemical Society, 77, 1028-32; 1955
   5. Huagong Shikan, 20(5), 30-32; 2006, U.S.S.R., 819119,07 Apr 1981
   6. US2658023 (1953); J. Am. Chem. Soc., 77, 763 (1955); JP Patent Publication (Kokoku) No. 46-20033 B (1971); JP Patent Publication (Kokoku) No. 47-13112 B (1972)
   7. J. Gen. Appl. Microbiol., 7, 113 (1961)
   8. Appl. Microbiol., 8,345 (1960)

Figure 3 is a view showing derivatives of hydrocorticoid and a synthetic method thereof. Mometasone: Trtrahedron, 55,3355 (1999); and Eur. Pat. Appl., 165037, 18 Dec 1985 Triamcinolone; J. Org. Chem., 59(24), 7534 (1994); and Eur. J. Appl. Microbiol. Biotechnol., 11, 81 (1981)

Dexamethasone; Eur. Pat. Appl., 165037, 18 Dec 1985

Budesonide; Huagong Shikan, 20(5), 20 (2006); and Huaxue Gongye Yu Gongcheng, 22(5), 354 (2005)

Fluticasone; JP Patent Publication (Kokai) No. 61-1699 A (1986); EP 165037 B1 ; EP 574318 B1; EP 1207166 A2; WO02/100878 A1; and EP 1526139 A1

Betamethazone: Tetrahedron Lett., 33, 4913 (1992)

## Claims

1. A protein described in any one of the following (a), (b) and (c):
(a) a protein consisting of the amino acid sequence shown in SEQ ID NO: 2;
(b) a protein consisting of an amino acid sequence comprising a deletion, substitution and/or addition of one or several amino acids with respect to the amino acid sequence shown in SEQ ID NO: 2, and having activity of cleaving the bond between positions 20 and 22 of a sterol side chain, wherein the maximum velocity (Vmax) used as a reaction rate parameter of the aforementioned activity is 50 mmol/min/mol or more; and
(c) a protein consisting of an amino acid sequence having homology of 95% or more with the amino acid sequence shown in SEQ ID NO: 2, and having activity of cleaving the bond between positions 20 and 22 of a sterol side chain, wherein the maximum velocity (Vmax) used as a reaction rate parameter of the aforementioned activity is 50 mmol/min/mol or more.

2. A sterol side chain cleavage enzyme having the following physicochemical properties:
(1) action: the enzyme acts on sterol represented by formula (I) as shown below and cleaves the carbon-carbon bond between positions 20 and 22 of sterol side chain portion by its activity of cleaving the bonds, so as to generate a compound represented by formula (II) as shown below;
(2) substrate specificity: when microorganisms that produce the enzyme are allowed to react with an aqueous solution containing 100 µg/ml 4-cholesten-3-one or cholesterol at 28°C for 5 hours, the conversion reaction rate from 4-cholesten-3-one to progesterone is 10% or more, and the conversion rate from cholesterol to pregnenolone is 10% or more;
(3) optimum pH: 7.5 to 8.0;
(4) optimum temperature for action: 15°C to 20°C;
(5) Thermostability: after preservation at 20°C for 140 hours, 30% or more of enzyme activity is maintained; and
(6) molecular weight: the putative molecular weight is assumed to be 53 to 54 KDa based on the amino acid sequence, and it is measured to be 50 to 56 KDa by SDS electrophoresis, 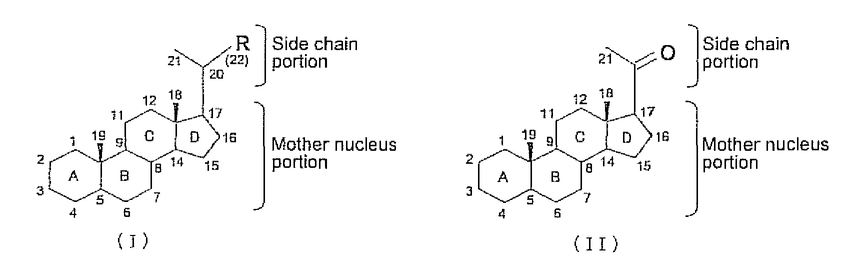 [wherein, in the formula (I),
the mother nucleus portion has a structure in which it consists of the A, B, C and D rings of a steroid, wherein
the mother nucleus portion has a carbon-carbon unsaturated bond(s) at zero site or at one or more sites of the positions 1 to 17 of the rings (except for the positions 10 and 13 thereof), and
carbon(s) at zero site or at one or more sites of the positions 1 to 19 of the rings (except for the positions 10 and 13 thereof) are independently substituted with
a group represented by the formula -OX
(namely, a hydroxy group wherein X = H; an acyloxyl group wherein X = CORₜ [wherein R₁ is a hydrogen atom, or an alkyl group, alkenyl group, alkynyl group or aromatic hydrocarbon containing 10 or less carbon atoms]; an O-alkyl group wherein X = R₂ [wherein R₂ is an alkyl group, alkenyl group or alkynyl group containing 10 or less carbon atoms, which may be optionally substituted with an oxygen atom]; a sulfate wherein X = SO₃M [wherein M is a hydrogen atom, an alkaline metal or an alkaline-earth metal]; an O-glycosyl group wherein X is the carbon at position 1 of a sugar; or an epoxy group wherein X is a carbon atom adjacent to said carbon), or
a keto group represented by the formula =O, and
in the side chain portion, R is a linear alkyl group, alkenyl group or alkynyl group containing 10 or less carbon atoms, which may have a cyclic portion, or a branched alkyl group, alkenyl group or alkynyl group containing 10 or less carbon atoms, which may have a cyclic portion,
the carbons at positions 20 and 21, and at zero site or at one or more sites in the R are independently substituted with
a group represented by the formula -OY
(namely, a hydroxy group wherein Y = H; an acyloxyl group wherein Y = COR₃ [wherein R₃ is a hydrogen atom, or an alkyl group, alkenyl group, alkynyl group or aromatic hydrocarbon containing 10 or less carbon atoms]; an O-alkyl group wherein Y = R₄ [wherein R₄ is an alkyl group, alkenyl group or alkynyl group containing 10 or less carbon atoms, which may be optionally substituted with an oxygen atom]; a sulfate wherein Y = SO₃M [wherein M is a hydrogen atom, an alkaline metal or an alkaline-earth metal]; an O-glycosyl group wherein Y is the carbon at position 1 of a sugar; or an epoxy group wherein Y is a carbon atom adjacent to said carbon), or
a keto group represented by the formula =O], and
[wherein, in the formula (II),
the mother nucleus portion has the same definitions as those of the mother nucleus portion of the formula (I), and
in the side chain portion, the carbon at position 21 is substituted with zero or one group represented by the formula -OY
(namely, a hydroxy group wherein Y = H; an acyloxyl group wherein Y = COR₃ [wherein R₃ is a hydrogen atom, or an alkyl group, alkenyl group, alkynyl group or aromatic hydrocarbon containing 10 or less carbon atoms]; an O-alkyl group wherein Y= R₄ [wherein R₄ is an alkyl group, alkenyl group or alkynyl group containing 10 or less carbon atoms, which may be optionally substituted with an oxygen atom]; a sulfate wherein Y = SO₃M [wherein M is a hydrogen atom, an alkaline metal or an alkaline-earth metal]; or an O-glycosyl group wherein Y is the carbon at position 1 of a sugar), or
zero or one keto group represented by the formula =O].

3. DNA encoding the protein according to claim 1.

4. DNA described in any one of the following (a), (b) and (c):
(a) DNA having the nucleotide sequence shown in SEQ ID NO: 1;
(b) DNA having a nucleotide sequence comprising a deletion, substitution and/or addition of one or several nucleotides with respect to the nucleotide sequence shown in SEQ ID NO: 1, and encoding a protein which has activity of cleaving the bond between positions 20 and 22 of a sterol side chain, wherein the maximum velocity (Vmax) used as a reaction rate parameter of the aforementioned activity is 50 mmol/min/mol or more; and
(c) DNA having a nucleotide sequence capable of hybridizing under stringent conditions with DNA having the nucleotide sequence shown in SEQ ID NO: 1 or a complementary sequence thereof, and encoding a protein which has activity of cleaving the bond between positions 20 and 22 of a sterol side chain, wherein the maximum velocity (Vmax) used as a reaction rate parameter of the aforementioned activity is 50 mmol/min/mol or more.

5. A fusion protein, wherein the enzyme protein according to claim 1 or 2, a ferredoxin protein having electron-transferring activity on the enzyme protein, and a ferredoxin reductase protein having electron-transferring activity on the ferredoxin protein are allowed to bind to one another, so that they can function as a single protein.

6. A recombinant vector comprising the DNA according to claim 3 or 4 and an expression control region capable of expression of a protein encoded by the DNA in a host cell.

7. A transformant formed by introducing the recombinant vector according to claim 6 into a host cell.

8. A method for producing a compound represented by formula (II) as shown below, which comprises allowing a sterol represented by formula (I) as shown below to come into contact with the following (i) or (ii):
(i) a mixture of the enzyme protein according to claim 1 or 2, a ferredoxin protein having electron-transferring activity on the enzyme protein, and a ferredoxin reductase protein having electron-transferring activity on the ferredoxin protein; or
(ii) the fusion protein according to claim 5, 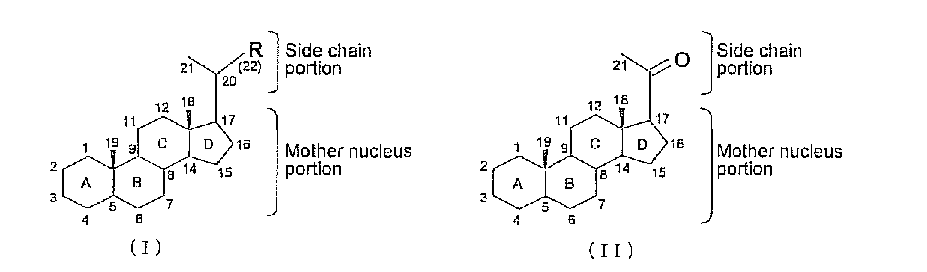 [wherein the definitions of the formula (I) and the formula (II) are the same as those of claim 1].

9. A method for producing a compound represented by formula (II) as shown below, which comprises allowing a sterol represented by formula (I) as shown below to come into contact with any one of the following (a) to (c):
(a) a mixture of a protein consisting of the amino acid sequence shown in SEQ ID NO: 23, a ferredoxin protein having electron-transferring activity on the aforementioned protein, and a ferredoxin reductase protein that transfers electron to the ferredoxin protein;
(b) a mixture of: a protein consisting of an amino acid sequence comprising a deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transferring activity on the aforementioned protein; and a ferredoxin reductase protein that transfers electron to the ferredoxin protein; and
(c) a fusion protein, wherein a protein consisting of the amino acid sequence shown in SEQ ID NO: 23 or a protein consisting of an amino acid sequence comprising a deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transferring activity on the enzyme protein; and a ferredoxin reductase protein having electron-transferring activity on the ferredoxin protein are allowed to bind to one another, so that they can function as a single protein, 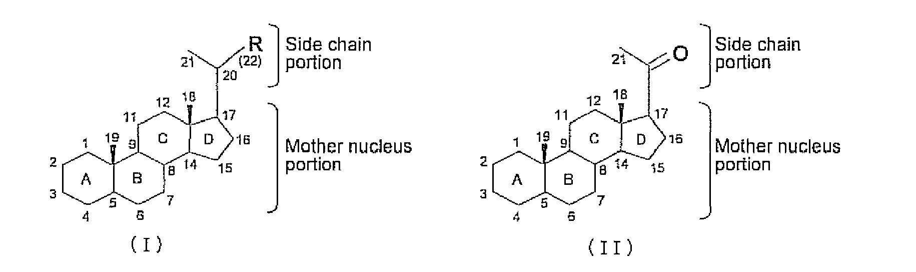 [wherein the definitions of the formula (I) and the formula (II) are the same as those of claim 1].

10. The method according to claim 8 or 9, which comprises allowing 3beta-hydoroxysteroid dehydrogenase and/or 3beta-hydroxysteroid:oxygen oxidoreductase, steroid 17α-hydroxylase, steroid 21-hydroaylase and steroid 11β-hydroxylase to further come into contact with the compound of the formula (II), so as to generate hydrocortisone.

11. The method according to any one of claims 8 to 10, wherein the sterol represented by the above formula (I) is cholesterol, 4-cholesten-3-one, 7-dehydrocholesterol, ergosterol, β-sitosterol, stigmasterol, campesterol, desmosterol, (20S)-20-hydroxycholest-4-en-3-one, (22R)-22-hydroxycholest-4-en-3-one, (20R,22R)-20,22-dihydroxycholest-4-en-3-one, or (20R,22S)-20,22-dihydroxycholest-4-en-3-one.

12. The method according to any one of claims 8 to 11, wherein the compound represented by the above formula (II) is pregnenolone, progesterone, or 7-dehydropregnenolone.

13. The method according to any one of claims 8 to 12, wherein the sterol represented by the above formula (I) is generated by allowing a raw material selected from among glucose, glycerin, methanol, ethanol, saccharose, acetic acid and citric acid, to come into contact with a yeast having ability to assimilate the raw material so as to generate the sterol represented by the above formula (I).

14. A method for producing hydrocortisone from a raw material selected from among glucose, glycerol, methanol, ethanol, sucrose, acetic acid and citric acid, wherein the method comprises culturing, in a medium containing the aforementioned raw material, a yeast having activity of generating a sterol represented by formula (1) as shown below from the aforementioned raw material, having activity of generating 3beta-hydroxysteroid dehydrogenase and/or 3beta-hydroxysteroid:oxygen oxidoreductase, steroid 17α-hydroxylase, steroid 21-hydroxylase and steroid 11β-hydroxylase, and also having any one of the following characteristics (A) to (E):
(A) having activity of generating a mixture of the enzyme protein according to claim 1 or 2, a ferredoxin protein having electron-transferring activity on the enzyme protein, and a ferredoxin reductase protein having electron-transferring activity on the ferredoxin protein;
(B) having activity of generating a mixture of a protein consisting of the amino acid sequence shown in SEQ ID NO: 23, a ferredoxin protein having electron-transferring activity on the aforementioned protein, and a ferredoxin reductase protein that transfers electron to the ferredoxin protein;
(C) having activity of generating a mixture of: a protein consisting of an amino acid sequence comprising deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transferring activity on the aforementioned protein; and a ferredoxin reductase protein that transfers electron to the ferredoxin protein;
(D) having activity of generating a fusion protein, wherein a protein consisting of the amino acid sequence shown in SEQ ID NO: 23 or a protein consisting of an amino acid sequence comprising a deletion, substitution and/or addition of one or several amino acids with respect to the amino acid sequence shown in SEQ ID NO: 23, and having activity of cleaving the bond between positions 20 and 22 of a sterol side chain, wherein the maximum velocity (Vmax) used as a reaction rate parameter of the aforementioned activity is 40 mmol/min/mol or more; a ferredoxin protein having electron-transferring activity on the enzyme protein; and a ferredoxin reductase protein having electron-transferring activity on the ferredoxin protein are allowed to bind to one another, so that they can function as a single protein; and
(E) having activity of generating a fusion protein, wherein the enzyme protein according to claim 1 or 2, a ferredoxin protein having electron-transferring activity on the enzyme protein, and a ferredoxin reductase protein having electron-transferring activity on the ferredoxin protein are allowed to bind to one another, so that they can function as a single protein, 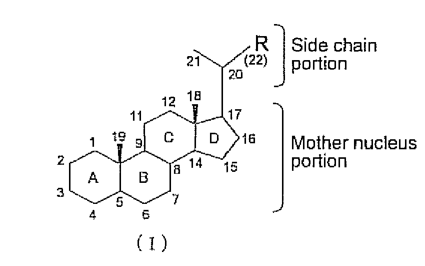 [wherein the definitions of the formula (I) are the same as those of claim 1].
